# EUROPEAN PATENT SPECIFICATION

(11) **EP 1 348 714 B1**
(45) Date of publication and mention of the grant of the patent: **03.01.2007**
(21) Application number: 02252133.0
(22) Date of filing: 25.03.2002
(51) Int. Cl.: C07K 14/705, A61K 38/17, A61P 11/06, A61P 37/08

(54) **Polypeptide useful as anti-allergic/antiasthmatic, methods for the preparation thereof and pharmaceutical compositions containing such polypeptide and their use**
Polypeptid als Antiallergikum/Antiasthmatikum, Methoden seiner Darstellung und pharmazeutische Zubereitungen, die ein solches Polypeptid enthalten, und ihre Verwendung
Polypeptide utile comme agent antiallergénique/antiasthmatique, son procédé de préparation et compositions pharmaceutique contenant ce peptide et leur utilisation

(43) Date of publication of application: 01.10.2003
(73) Proprietor: COUNCIL OF SCIENTIFIC AND INDUSTRIAL RESEARCH, New Delhi 100 001 (IN)
(72) Inventor: Kundu, Bijoy, Uttar Pradesh (IN); Khare, Sanjay Kumar, Uttar Pradesh (IN); Singh, Rashmi, Uttar Pradesh (IN); Nath, Amar, Uttar Pradesh (IN); Gupta, Prem Prakash, Uttar Pradesh (IN); Agarwal, Kamlesh Chandra, Uttar Pradesh (IN); Dwivedi, Anil Kumar, Uttar Pradesh (IN); Singh, Satyawan, Uttar Pradesh (IN)
(74) Representative: Manaton, Ross Timothy

(56) References cited:
- KUNDU B ET AL.: "Antiallergic activity of novel hexapeptides related to immunoglobuline E" PROTEIN AND PEPTIDE LETTERS, vol. 6, no. 6, 1999, pages 379-384, XP001074382
- WEINSTEIN B: "Chemistry and biochemistry of amino acids, peptides and proteins" 1982 , MARCEL DEKKER, INC. , NEW YORK AND BASEL XP002032461 * page 338 *
- VERMEHREN C ET AL.: "Time dependent effects of two absorption enhancers on the nasal absorption of growth hormone in rabbits" INTERNATIONAL JOURNAL OF PHARMACEUTICS, vol. 128, 1996, pages 239-250, XP002213867
- NOGUCHI K ET AL.: "Synthesis of Peptides Related to Immunoglobulin E (IgE) and the Examination of Their Pharmaceutical Activity" CHEMICAL AND PHARMACEUTICAL BULLETIN, vol. 38, no. 9, September 1990 (1990-09), pages 2463-2466, XP001073996

### SEQUENCE LISTING

<110> Council of scientific and Industrial Research
<120> Polypeptide useful As Anti-Allergic/Antiasthmatic, Methods For The prepartion Thereof, Pharmaceutical compositions containing such Polypeptide And use Thereof
<130> RTM NF9/97 EP
<140> EP 02252133.0
   <141> 2002-03-25
<160> 11
<170> PatentIn version 3.1
<210> 1
   <211> 6
   <212> PRT
   <213> Artificial
<220>
   <221> MISC_FEATURE
   <222> (2)..(2)
   <223> MeGly, sarcosine
<220>
   <221> MOD_RES
   <222> (6)..(6)
   <223> Hyl
<400> 1
   Ala Xaa Gly Asp Gly Lys
   1               5
<210> 2
   <211> 6
   <212> PRT
   <213> Artificial
<220>
   <221> MOD_RES
   <222> (1) .. (1)
   <223> N-Me-Ala
<220>
   <221> MISC_FEATURE
   <222> (3)..(3)
   <223> MeGly, sarcosine
<220>
   <221> MOD_RES
   <222> (6)..(6)
   <223> Hyl
<400> 2
   Ala Gly Xaa Asp Gly Lys
   1               5
<210> 3
   <211> 6
   <212> PRT
   <213> Artificial
<220>
   <221> MOD_RES
   <222> (6)..(6)
   <223> Hyl
<220>
   <221> MISC_FEATURE
   <222> (2)..(3)
   <223> MeGly, sarcosine
<400> 3
   Ala Xaa Xaa Asp Gly Lys
   1               5
<210> 4
   <211> 6
   <212> PRT
   <213> Artificial
<220>
   <221> MOD_RES
   <222> (1)..(1)
   <223> N-allylalanine
<220>
   <221> MISC_FEATURE
   <222> (3)..(3)
   <223> MeGly, sarcosine
<220>
   <221> MISC_FEATURE
   <222> (5)..(5)
   <223> MeGly, sarcosine
<220>
   <221> MOD_RES
   <222> (6)..(6)
   <223> Hyl
<400> 4
   Ala Gly Xaa Asp Xaa Lys
   1               5
<210> 5
   <211> 6
   <212> PRT
   <213> Artificial
<220>
   <221> MISC_FEATURE
   <222> (2)..(2)
   <223> MeGly, sarcosine
<220>
   <221> MOD_RES
   <222> (6)..(6)
   <223> Hyl
<220>
   <221> MISC_FEATURE
   <222> (5)..(5)
   <223> MeGly, sarcosine
<400> 5
   Ala Xaa Gly Asp Xaa Lys
   1               5
<210> 6
   <211> 6
   <212> PRT
   <213> Artificial
<220>
   <221> MISC_FEATURE
   <222> (5)..(5)
   <223> MeGly, sarcosine
<220>
   <221> MOD_RES
   <222> (6)..(6)
   <223> Lys1-amine
<400> 6
   Ala Gly Gly Asp Xaa Lys
   1               5
<210> 7
   <211> 6
   <212> PRT
   <213> Artificial
<220>
   <221> MISC_FEATURE
   <222> (3)..(3)
   <223> MeGly, sarcosine
<220>
   <221> MOD_RES
   <222> (6)..(6)
   <223> Lysl-NHPr(n)
<220>
   <221> MISC_FEATURE
   <222> (5)..(5)
   <223> MeGly, sarcosine
<400> 7
   Ala Gly Xaa Asp Xaa Lys
   1               5
<210> 8
   <211> 6
   <212> PRT
   <213> Artificial
<220>
   <221> MISC_FEATURE
   <222> (5)..(5)
   <223> MeGly, sarcosine
<220>
   <221> MOD_RES
   <222> (6)..(6)
   <223> Lys-amine
<400> 8
   Ala Gly Gly Asp Xaa Lys
   1               5
<210> 9
   <211> 6
   <212> PRT
   <213> Artificial
<220>
   <221> MISC_FEATURE
   <222> (5)..(5)
   <223> MeGly, sarcosine
<220>
   <221> MOD_RES
   <222> (6)..(6)
   <223> Hyl
<400> 9
   Ala Gly Gly Asp Xaa Lys
   1               5
<210> 10
   <211> 6
   <212> PRT
   <213> Artificial
<220>
   <221> MOD_RES
   <222> (1)..(1)
   <223> N-allylalanine
<220>
   <221> MISC_FEATURE
   <222> (2)..(2)
   <223> MeGly, sarcosine
<220>
   <221> MOD_RES
   <222> (6)..(6)
   <223> Hyl
<400> 10
   Ala Xaa Gly Asp Gly Lys
   1               5
<210> 11
   <211> 6
   <212> PRT
   <213> Artificial
<220>
   <221> MOD_RES
   <222> (2)..(2)
   <223> N-allylglycine
<220>
   <221> MOD_RES
   <222> (6)..(6)
   <223> Lysyl-amide
<400> 11
   Ala Gly Gly Asp Gly Lys
   1               5

## Description

### Field of the invention

This present invention relates to new peptides L-N-substituted- alanyl-N-substituted-glycyl-N-substituted-glycyl-L-aspartyl-N-substituted-glycyl-L-lysyl derivatives which can be used as therapeutic agents for allergy/asthma and a process for preparing the said compounds and its formulation for administration by nasal route.

The present invention particularly relates to L-N-substituted-alanyl-N-substituted-glycyl-N-substituted-glycyl-L-aspartyl-L-N-substituted-glycyl-L-lysyl derivatives, process for preparing the said compounds and to their use in medicine.

### Background of the invention

Asthma is a complex disorder and its occurrence has almost doubled worldwide in the last twenty years. This may be attributed to worldwide increase in environmental pollutants and allergens, and as a result, greater human exposure to viral respiratory infections. In the United States alone, there are 5000 deaths each year and the rate continues to increase. In recent years, important advances have been made in the development of better symptomatic and pallative therapy for asthma. They are novel leukotriene antagonists, phosphodiesterase inhibitors, long acting bronchodilators, corticosteroids, mediator antagonist. However, these agents are known to simply provide symptomatic relief of asthma and do not control inflammation. Beside this, these drugs are associated with several undesired side effects.

Several structurally diverse peptides have also been reported with antiallergic activity. In this context, the polypeptides L-N-substituted-alanyl-N-substituted-glycyl-N-substituted glycyl-L-aspartyl-N-substituted glycyl-L-lysyl derivatives of formula 1 are structurally novel compounds and show significant antiallergic/antiasthmatic activity. Thus these compounds would be useful in the treatment of allergy disorders.

The most commonly used antiallergic drugs are disodium cromoglycate (DSCG), nedocromil sodium, amtexanox, repirinast, tazanolast, and pemirolast potassium. Even though DSCG is being used for 30 years, clinically it is an enigma because it is effective in some patients and yet in other, apparently similar patients it affords little protection. Further repeated administration of DSCG has been found to exhibit tachyphlaxis.

Advances in biotechnology have made available a large number of protein and peptide drugs for the treatment of a variety of diseases. These drugs are unsuitable for oral administration because they are significantly degraded in the gastrointestinal tract or considerably metabolized by first-pass effect in the liver. Even the parental route is inconvenient for long term therapy. Of many alternate routes tried, intranasal drug delivery is found much promising for administration of these drugs. Systemic absorption from nasal cavity has been described for several drugs including scopolamine, hydralazine, propranolol, insulin, butorphanol, enkephalins, buprenophine, dobutamine, human growth hormone (hGH), calcitonin, luteinizing hormone-releasing hormone (LHRH) and estradiol.

Cyclodextrins are reported in the literature that they increase water solubility, dissolution, bioavailability and stability of compound by forming inclusion complexes. [Z. Shao, R. Krishnamoorthy and A. K. Mitra, Pharm. Res., 9: 1157-1163 (1992)]. Recently it was reported in the literature that beta-Cyclodextrins increased the half life of leucine enkephaline, a peptide, from 44 min. to 75 min. in case of enzymatic hydrolysis with leucine amino-peptidase (W. J. Irwin, A. K. Dwivedi, P.A. Holbrook and M. J. Dey, Pharm. Res. 11, 1698-1703, 1994). Uekama et al. (Drug Targeting Delivery 3, 1994, 411-456) as part of a larger review has recently reviewed the use of Cyclodextrins in nasal drug delivery. Like ophthalmic drug deliver, nasal delivery may benefit from the presence of cyclodextrins by changes in nasal mucosa permeability, enhanced drug solubility or a change in the metabolism rate of the drug at the site of delivery. Balanced against these possible positive effects are possible concerns with nasal ciliary damage that could lead to long term toxicity questions. For example, high dimethyl beta cyclodextrin doses have been shown to adversely affect the nasal mucosa in both in vitro and some *in vivo* experiments. However, it was much less damaging than the surfactants, sodium glycocholate and laureth-9, and the phospholipid, L-a-lysophosphatidylcholine (E. Marttin, J. C. Verhoef, S. G. Romeijn and F. W. H. M. Merkus, Pharma. Res. 12, 1995, 1151-1157). Neverthless, many researchers have focused on the use of dimethyl beta cyclodextrin for nasal delivery of a number of agents even though some results suggest potential changes in nasal membranes occur at high levels of exposure to this cyclodextrin derivative. The major focus of these studies is the use of dimethyl beta cyclodextrin to enhance the delivery of various steroids, proteins and peptides (Uekama et al. Drug Targeting Delivery 3, 1994, 411-456, W. A. J. Hermens, European J. of Obstetrics and Gynecology and Reproductive Biology, 43, 1992, 65-70, E. Marttin, J. C. Verhoef, S. G. Romeijn and F. W. H. M. Merkus,, In Proceedings of the 8th International Symposium on cyclodextrins, Kluwer Academic Publishers, Dordrecht, 1996, 381-386).

### Prior art

Among a large number of the molecules belonging to peptides and showing antiallergic activity, some relevant ones are:
1. Cyclic hexapeptides of formula Cyclo (Gly-Lys-Ala-βAsp-Ser-βAsp) (JP 06,336,496; 1993);
2. Repetitive units of pentapeptide of formula Asp-Ser-Asp-Gly-Lys (JP 04,187,088; 1990);
3. Polymeric pentapeptide of formula Asp-Ser-Asp-Glu-Lys (JP 04,187,088; 1990);
4. Undecapeptide of formula Arg-Pro-Lys-Pro-Gln-Gln-Phe-Phe-Gly-Leu-Met-NH₂ (WO 92,20,360; 1991; 1991);
5. Hexapeptides of formula A-B-L(or D)-Pro-C-D-E [A = L or D- form of deaminoarginine or N^{α}-deaminolysine; B = L- or D-form of Arg, Lys or His; C = L- or D-form of Tyr, Trp or Phe; L- or D- form of Val, Ile or Leu; E = L- or D-form of Val, Ile, one of H atoms of the amino group may be substituted with a C₁₋₄-alkyl group and the C-terminal carboxyl group may be substituted with CO₂R*(R* = C₁₋₄-alkyl), CH₂OR or CONHR where R = H or C₁₄-alkyl] (EP 526 192; 1991);
6. Amino acid amides and dipeptides of formula R²NHCH(CH₂XR¹)CONHCHR³R⁴, (X = O, S; R¹ = alkyl; R² = H, CO₂H, alkyloxycarbonyl, aryloxycarbonyl; R⁴ = H, alkyl, aralkyl, heteroarylalkyl, hydrocyalkyl, thioalky, alkylthioalkyl, aminoalkyl, carboxyalkyl, carbamoyl, guanidinoalkyl, or sulfoalkyl), (PCT WO 93 21,211; 1992);
7. N⁵-substituted-glutamines of formula XNHCH(CO₂H)CH₂CH₂CONH(CH₂)ₙRACO₂H, (X = H, Ac; R = alkylene, phenylene; A = direct bond, alkylene, CH:CH; n = 0, 1), (JP 06, 172287, 1992);
8. Peptide as specific inhibitor of IgE antibody of formula (JP 06,239,887, 1993); 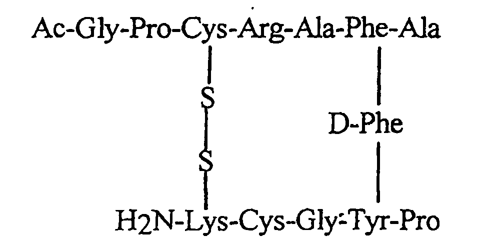
9. Peptides derived from RGD sequence of formula Arg-Gly-Asp-Ser (PCT Int. Appl. 95 13,826; 199; 1993).
10. Hexapeptides corresponding to IgE Fc region (330-334) show antiallergic activity (Kundu et al., 1999, Protein and Peptide Letters 6 : 379-384).

### Objects of the invention

The main object of the invention is to provide novel peptides that exhibit better therapeutic efficacy to treat allergy/asthma over the existing antiallergic/antiasthmatic drugs.

It is another object of the invention to provide novel L-N-substituted-alanyl-N-substituted-glycyl-N-substituted -glycyl-L-aspartyl-N-substituted -glycyl-L-lysyl derivatives exhibiting activity against allergy/asthma.

It is a further object of the invention to provide a process for preparing L-N-substituted-alanyl-N-substituted-glycyl-N-substituted- glycyl-L-aspartyl-N-substituted - glycyl-L-lysyl derivatives.

It is yet another object of the invention to provide a pharmaceutical composition comprising hexapeptides and pharmaceutical acceptable additive(s) and a process for preparing such composition.

It is another object of the invention to provide a method of treating allergy/asthma and related disorders in patients such as human being and mammals.

### Summary of the invention

These and other objects of the present invention are achieved by the novel compounds of formula 1 below.

Accordingly, the present invention provides a polypeptide of formula 1: 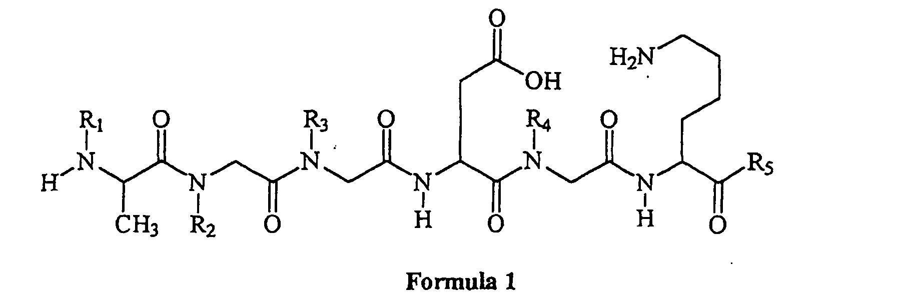 wherein R¹, R², R³, R⁴ are selected from the group consisting of H, CH₃ and CH₂=CH₂-CH₂ and R⁵ is selected from the group consisting of OH or NH₂ and NHCₙH₂ₙ₊₁ (n = 1 to 18), characterised in that the polypeptide contains unsubstituted amino acid residues and substituted amino acid residues, wherein each such substitution consists of either a methyl or an allyl group.

In a preferred embodiment, the polypeptide is a hexapeptide selected from the group consisting of:
(a) Ala-Sar-Gly-Asp-Gly-Lys-OH
(b) N-MeAla-Gly-Sar-Asp-Gly-Lys-OH
(c) Ala-Sar-Sar-Asp-Gly-Lys_OH
(d) N-allylAla-Gly-Sar-Asp-Sar-Lys-OH
(e) Ala-Sar-Gly-Asp-Sar-Lys-OH
(f) Ala-Gly-Gly-Asp-Sar-Lys-NH₂
(g) Ala-Gly-Sar-Asp-Sar-Lys-NHPr(n)
(h) Ala-Gly-Gly-Asp-Sar-Lys-NH₂
(i) Ala-Gly-Gly-Asp-Sar-Lys-OH

A preferred group of compound comprises those in which R¹ = H; R² = CH_{3;} R³ = H; R⁴= CH₃ and R⁵ is OH or amide or amide group substituted with aliphatic chains, CₙH₂ₙ₊₁ (n=1 to 18).

The invention also provides a process for the preparation of a polypeptide of formula 1: 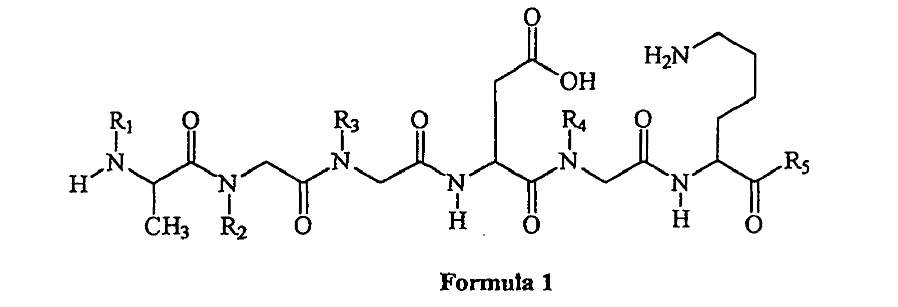 wherein R¹, R², R³, R⁴ are selected from the group consisting of H, CH₃ and CH₂=CH₂-CH₂ and R⁵ is selected from the group consisting of OH or NH₂ and NHCₙH₂ₙ₊₁ (n = 1 to 18), comprising condensing suitably protected amino acids and substituted amino acids wherein the substituted amino acids includes either methyl or allyl group in the presence of one of the c-terminal derivative selected from the group of OH or NH₂ or long chain aliphatic amines of the formula NHCₙH₂ₙ₊₁ (n = 1 to 18) and coupling reagents and organic solvent ranging from temperatures 0 to 60°C for between 3 hrs to 72 hrs to produce the corresponding polypeptide of formula 1.

In another embodiment of the invention, the synthesis of the intermediate fragments: dipeptide or tripeptide or tetrapeptide includes reaction of suitably derivatized N-protected amino acids or N-substituted amino acids and suitably derivatized C-protected amino acids or N-substituted amino acids in organic solvents in presence of coupling reagents at temperatures ranging from 0° to 60° C for between 3 hrs to 72 hrs.

In another embodiment of the invention, the fragments selected are N-terminal tripeptide and C-terminal tripeptide.

In another embodiment of the invention, hydroxybenzotriazole or p-nitrophenol or N-hydroxysuccinimide is included as additives during condensation.

In another embodiment of the invention, the molar ratio of the intermediate fragments and amino acid derivatives are 1:1 in solution phase.

In another embodiment of the invention, the molar ratio in solid phase of N-protected amino acids to resin bound amine is 1: 2.5 to 10 folds.

In yet another embodiment of the invention the organic solvent is selected from the group consisting of DMF, DCM and NMP.

In another embodiment of the invention, removal of N-protection is done using acids selected from TFA or 10-50% (v/v) HCl/dioxane.

In another embodiment of the invention, removal of N-protection is done using bases selected from the group consisting of piperidine, DBU, DABCO and pyridine.

In another embodiment of the invention, the process is carried out in either solution phase or solid phase.

In a further embodiment of the invention, in solution phase, synthesis is carried out by condensing N-terminal tripeptide fragment with C-terminal fragment.

In another embodiment of the invention, a solid support having a compatible reactive functional group is used selected from polyamide or polystyrene bared with suitable linking agents such as 4-alkoxy benzyl alcohol or Rink amide resin.

In a further embodiment of the invention, (i) C-terminal activated N-protected lysine is anchored onto a solid support having a compatible reactive functional groups; (ii) the N-protecting group of the anchored lysine obtained in (i) are deprotected; (iii) N-protected C-terminal activated sarcosin is cooupled onto the deprotected amino group of lysine obtained in step (iii); (iv) (ii) and (iii) of deprotecting and coupling respectively are repeated sequentially with aminoacids to obtain a solid support attached polypeptide having the sequence L'-alanyl-glycyl-glycyl-L-gsparlyl-sarcosyl-L-lysysl; (v) the polypeptide from the solid support is cleaved to obtain compound of formula 1.

The invention also relates to pharmaceutical compositions comprising a polypeptide L-N-substituted-alanyl-N-substituted-glycyl-N-substituted-glycyl-L-aspartyl-N-substituted-glycyl-L-lysyl derivative of formula 1: 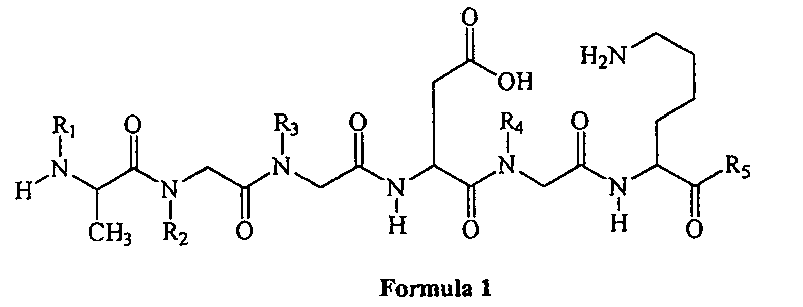 wherein R¹, R², R³, R⁴ are selected from the group consisting of H, CH₃ and CH₂=CH₂-CH₂ and R⁵ is selected from the group consisting of OH or NH₂ and NHCₙH₂ₙ₊₁ (n = 1 to 18), in admixture with a pharmaceutically acceptable carrier, characterised in that the polypeptide contains unsubstituted amino acid residues and substituted amino acid residues, wherein each such substitution consists of either a methyl or an allyl group.

The invention further relates to a process for preparing the pharmaceutical composition by bringing the compound into association with a pharmaceutically acceptable additive.

The present invention also relates to a process for converting a polypeptide of formula 1: 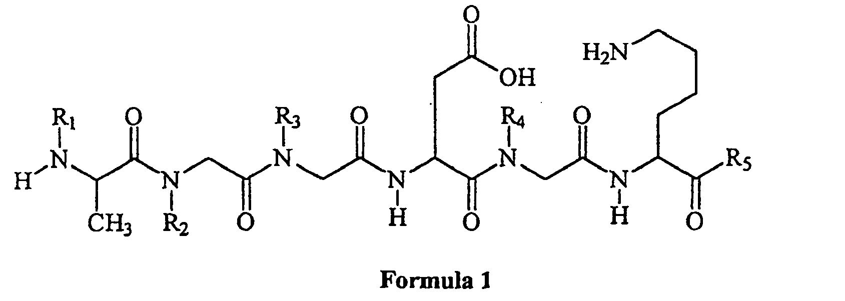 wherein R¹, R², R³, R⁴ are selected from the group consisting of H. CH₃ and CH₂=CH₂-CH₂ and R⁵ is selected from the group consisting of OH or NH₂ and NHCₙH₂ₙ₊₁ (n = 1 to 18), to a pharmaceutically acceptable formulation, comprising preparing solution of said peptide and cyclodextrin in a protic solvent separately, mixing the above said solutions at a temperature in the range of 10 to 80° C to make a clear solution, removing the solvent to get a free flowing complex , mixing the complex so obtained in a vehicle to get the said formulation, characterised in that the polypeptide contains unsubstituted amino acid residues and substituted amino acid residues, wherein each such substitution consists of either a methyl or an allyl group.

In one embodiment of the invention, the cyclodextrin used is selected from the group consisting of naturally occurring alpha-cyclodextrin, beta-cyclodextrin, gama-cyclodextrin and their derivatives selected in turn from the group consisting of dimethyl beta-cyclodextrin and hydroxy propyl beta-cyclodextrin.

In another embodiment of the invention, the solvent is removed by freeze drying, spray drying, coprecipitation or solvent evaporation.

In another embodiment of the invention, the vehicle used is selected from the group consisting of 0.2 M phosphate buffer solution of pH 6.5 containing sodium chloride and methyl cellulose, and a mixture of alcohol and commercial propellant.

In another embodiment of the invention, the amount of hexapeptide used ranges from 5 to 40 % by weight of the inclusion complex [1:5 to 1:1].

In another embodiment of the invention, the formulation is made in the form of nasal drops/spray.

The invention also relates to method of treating allergy/asthma disorders with the pharmaceutical composition of the present invention.

### DETAILED DESCRIPTION OF THE INVENTION

The present invention provides compounds of formula 1 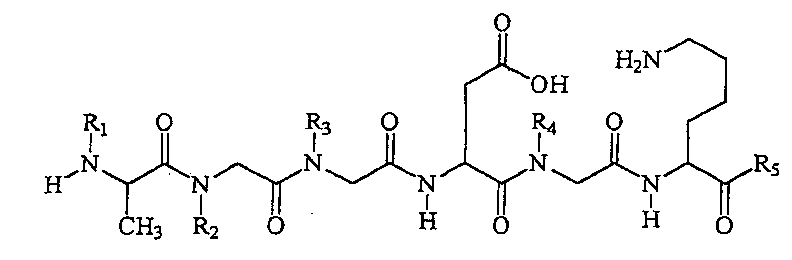 wherein R¹, R², R³, R⁴ represents H or CH₃ or CH₂=CH₂-CH₂ and R⁵ may be either OH or NH₂ or NHCₙH₂ₙ₊₁ (n = 1 to 18), characterised in that the polypeptide contains unsubstituted amino acid residues and substituted amino acid residues, wherein each such substitution consists of either a methyl or an allyl group.

The compounds of the invention have shown to possess high order of antiallergic activity by *i.p., p.o.* and nasal route of administration. They were found to be at least 50 times more active than standard drug DSCG dose per dose. The major drawback of DSCG is that, it can not be given by oral route. The compound also exhibited potent antiasthmatic activity using aerosol test.

A method of preparation of the inventive compounds starts from the condensation of suitably protected amino acids on solid phase followed by deprotection and cleavage of protecting groups in one step to get the desired compounds of formula

Another method involves fragment condensation (2 + 4), (3 + 3), (1 + 5) or assembly of peptides in a step wise manner starting from C-terminal in solution phase.

The compounds of the present invention can be used for the preparation of nasal drops/spray by conventional methods useful as therapeutic agent. These formulations are used to produce antiallergic/antiasthmatic activity and contain effective compounds useful in the method of the invention. The most preferred compound of the invention is L-alanyl-glycyl-glycyl-L-aspartyl-sar-L-1ys (96/199).
Mainly the present invention centers around the following objects:
i. The first object of the invention is to provide novel peptides that exhibit better therapeutic efficacy to treat allergy/asthma over the existing antiallergic/antiasthmatic drugs.
ii. The second object of the invention is to provide novel L-N-substituted-alanyl-N-substituted-glycyl-N-substituted -glycyl-L-aspartyl-N-substituted -glycyl-L-lysyl derivatives exhibiting activity against allergy/asthma.
iii. The third object of the invention is to provide a process for preparing L-N-substituted-alanyl-N-substituted-glycyl-N-substituted- glycyl-L-aspartyl-N-substituted -glycyl-L-lysyl derivatives.
iv. The fourth object of the invention relates to a pharmaceutical composition comprising hexapeptides and pharmaceutical acceptable additive(s) and a process for preparing such composition.
v. The fifth object of the invention relates to a method of treating allergy/asthma and related disorders in patients such as human being and mammals.

To achieve the above and other objects, the present invention provides novel pharmacologically active substances, specifically new L-N-substituted-alanyl-N-substituted-glycyl-N-substituted glycyl-L-aspartyl-N-substituted -glycyl-L-lysyl derivatives which are used as potential therapeutic agents for allergic/asthma disorders.

Accordingly, the invention provides novel compounds of formula 1. 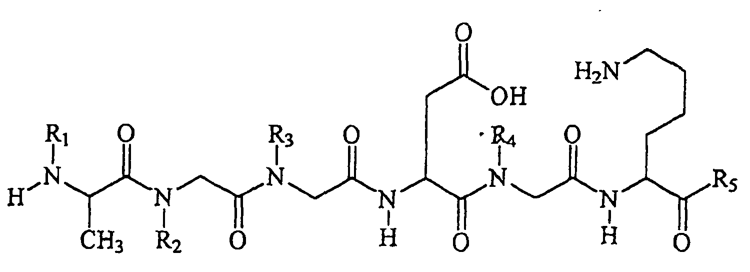 wherein R¹, R², R³, R⁴ represents H or CH₃ or CH₂=CH₂-CH₂ and R⁵ may be either OH or NH₂ or NHCₙH₂ₙ₊₁ (n = 1 to 18) characterised in that the polypeptide contains unsubstituted amino acid residues and substituted amino acid residues, wherein each such substitution consists of either a methyl or an allyl group. Preferred compounds are as follows:
1. Ala-Sar-Gly-Asp-Gly-Lys-OH
2. N-MeAla-Gly-Sar-Asp-Gly-Lys-OH
3. Ala-Sar-Sar-Asp-Gly-Lys_OH
4. N-allylAla-Gly-Sar-Asp-Gly-Lys-OH
5. Ala-Sar-Gly-Asp-Sar-Lys-OH
6. Ala-Gly-Gly-Asp-Sar-Lys-NH₂
7. Ala-Gly-Sar-Asp-Sar-Lys-NHPr(n)
8. Ala-Gly-Gly-Asp-Sar-Lys-NH₂
9. Ala-Gly-Gly-Asp-Sar-Lys-OH

In the specification and claims, the compounds with R¹, R², R³, R⁴ = H designates amino acids while compounds with R¹, R², R³, R⁴ = CH₃ designates N-methyl amino acids. R⁵ designates either OH or NH₂ or substituted amides NHCₙH₂ₙ₊₁ (n = 1 to 18).

A preferred group of compound comprises those in which R¹ = H; R² = CH_{3;} R³ = H; R⁴= CH₃ and R⁵ is OH or amide or amide group substituted with aliphatic chains. The compounds of this invention have useful biological activities and have in particular strong antiallergic/antiasthmatic activity.

The invention also provides a pharmaceutical composition comprising a compound of formula 1 in admixture with a pharmaceutically acceptable conventional carriers and a process for the preparation of a pharmaceutical composition which comprises bringing a compound of the formula 1 into association with a pharmaceutically acceptable conventional carrier.

In addition, the invention provides a method of treating allergy/asthma in mammals, by administering to a subject in need thereof an effective amount of a compound of formula 1.

The reaction sequence leading to L-N-substituted-alanyl-N-substituted-glycyl-N-substituted glycyl-L-aspartyl-N-substituted glycyl-L-lysyl derivatives is shown in scheme 1 below:

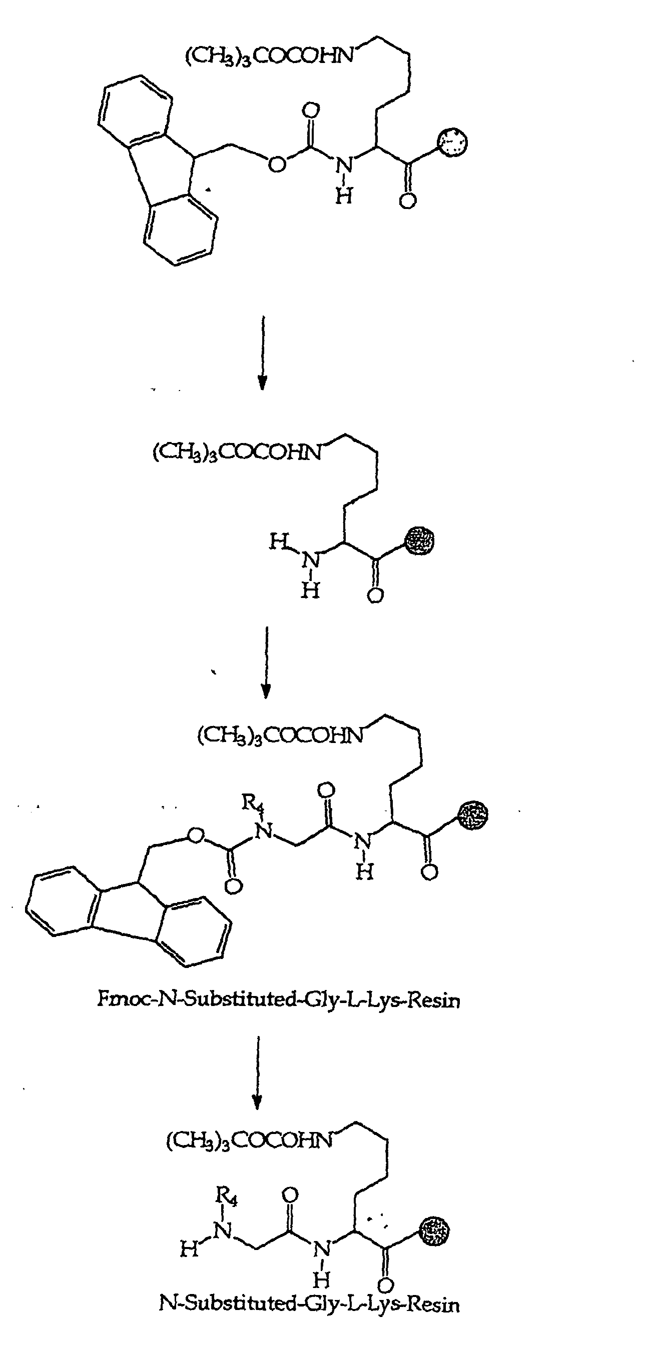

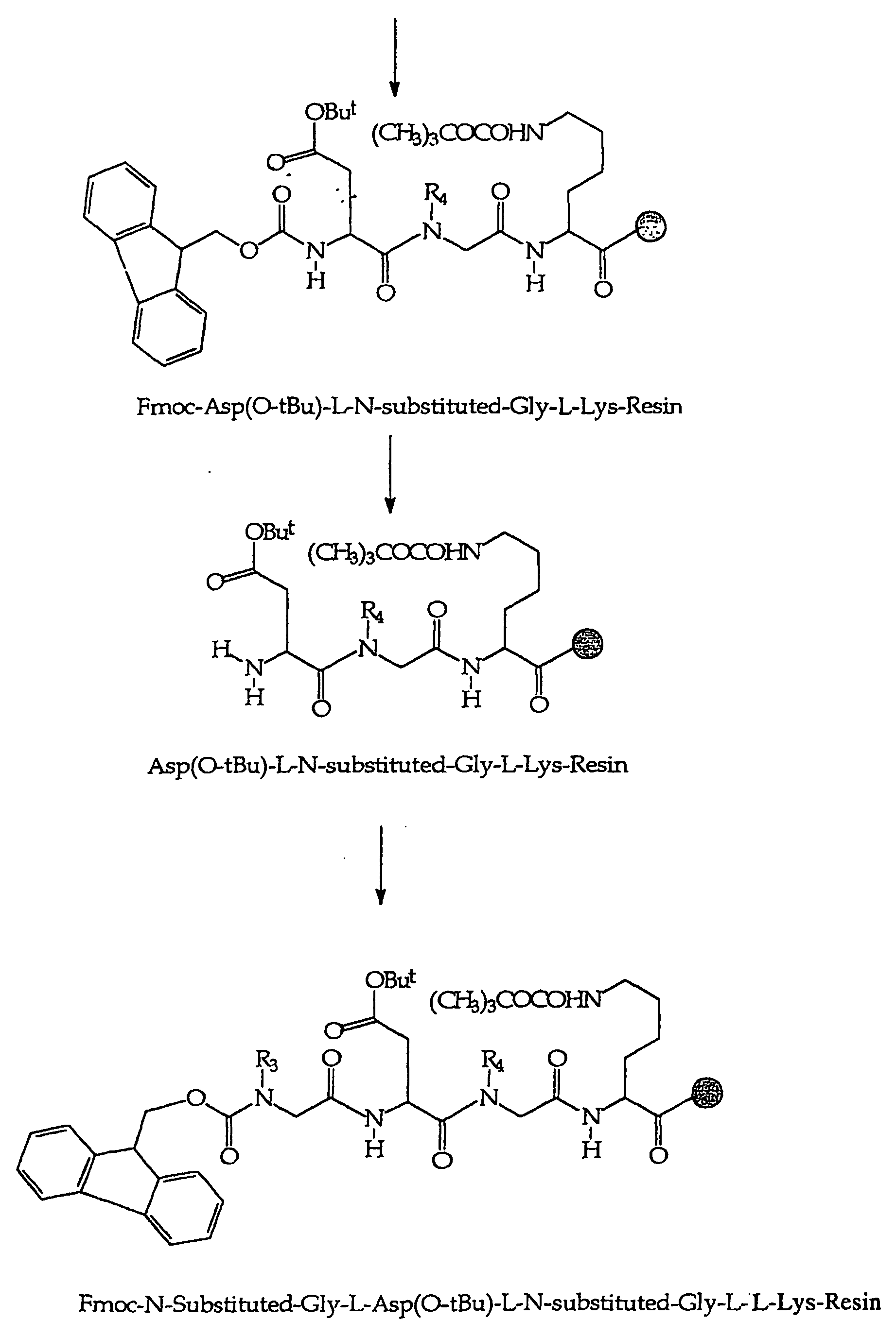

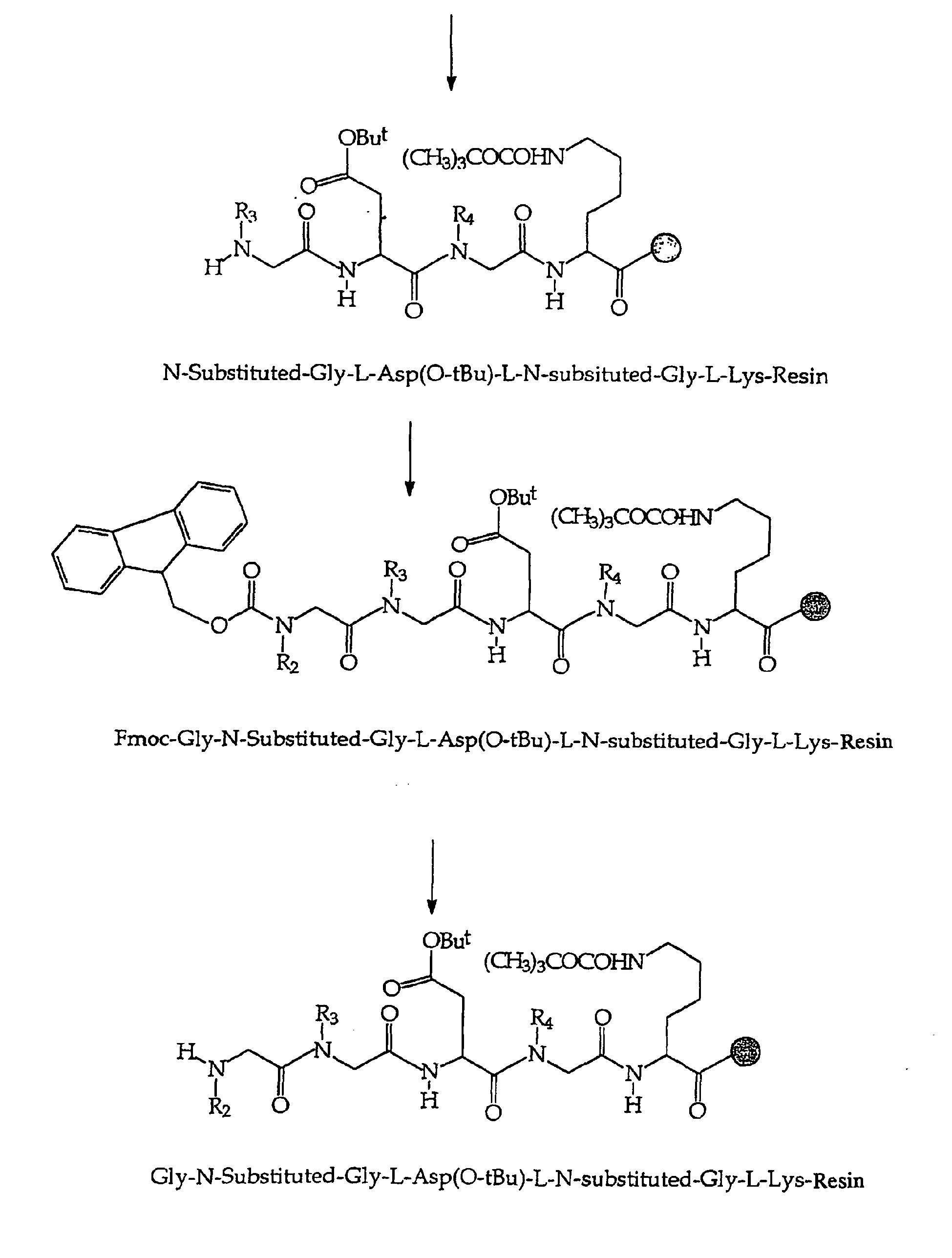

As can be seen according to the foregoing scheme, there are two methods leading to the synthesis of compounds of formula 1.

In the first method the peptide derivatives of formula 1 can be synthesized using solid phase method. The solid support having a compatible reactive functional group used may be polyamide or polystyrene based with suitable linking agents such as Wang's resin, Rink Amide AM resin, Merrifield resin, Sieber amide resin etc. For the protection of amino function in amino acids any one of the groups such as t-butyloxy carbonyl or 9-fluorenylmethoxy carbonyl can be employed. For side chain protection of the carboxyl function of Aspartic acid, t-butyl and for the side chain protection of the amino function of Lysine, t-butyloxy carbonyl or 9-fluorenylmethoxy carbonyl can be employed. For amide bond formation one of the coupling reagents such as N,N-dicycloisopropyl carbodiimide (DIC), 1-hydroxy-benzotriazole (HOBt), N,N-dicyclohexylcarbodiimide (DCC), Benzotriazole-1-yl-oxy-tris-(dimethylamino)- phosphonium hexafluorophosphate (BOP) and Benzotriazole-1 -yl-oxytripyrrplidenephosphonium hexafluorophosphate (PyBOP) can be employed. For the removal of protecting group at every step either piperidine or TFA can be used. The reaction may be carried out in solvents selected from dimethylformamide, methylene chloride, N-methylpiperidone, using bases triethylamine, diisopropylamine, pyridine, N-methylmorpholine etc to improve the yield. Finally the peptide can be cleaved from the resin using trifluoroacetic acid, trifluoromethanesulfonic acid and HF in presence of cocktail of scavengers such as thiophenol, ethanedithiol anisole, thioanisole etc.

In the second method compounds of formula 1 have been synthesized by solution phase as shown in scheme 2 of the accompanied drawings. Synthesis was carried out using 3 + 3 fragment condensation strategy. For the protection of amino function in amino acids any one of the protecting groups such as t-butyloxy carbonyl or 9-fluorenylmethoxy carbonyl can be employed. For side chain protection of the carboxyl function of Aspartic acid, benzyl group and for the side chain protection of the amino function of Lysine, t-butyloxy carbonyl or 9-fluorenylmethoxy carbonyl can be employed. For the protection of α-carboxyl group any one of the groups can be employed: benzyl ester or amide or substituted amides. For amide bond formation one of the coupling reagents such as N,N-dicycloisopropyl carbodiimide (DIC), 1-hydroxy-benzotriazole (HOBt), N,N-dicyclohexylcarbodiimide (DCC), Benzotriazole-1-yl-oxy-tris-(dimethylamino) -phospho- nium hexafluorophosphate (BOP) and Benzotriazole-1 -yl-oxytripyrrplidenephosphonium hexafluorophosphate (PyBOP) can be employed. For the removal of protecting group at every step either piperidine or TFA can be used. The reaction may be carried out insolvents selected from dimethylformamide, methylene chloride, N-methylpiperidone, using bases triethylamine, diisopropylamine, pyridine, N-methylmorpholine etc to improve the yield. Finally the free peptide can be obtained by catalytic hydrogenation using one of solvents such as methanol, ethanol, hydrazine etc

L-N-substituted-alanyl-N-substituted-glycyl-N-substituted-glycyl-L-aspartyl- N-substituted glycyl-L-lysyl derivatives in free form can, if desired be converted in to their nontoxic pharmaceutically acceptable acid salts. The acid salt which may be formed comprise, for example, salts with inorganic acids such as hydrochloride, hydrobromide, hydroiodide. They may also comprise salts with organic acids including monobasic acids such as acetate formate etc.

The compounds of invention show marked antiallergic/antiasthmatic activity.

### Pharmacological activity

The interaction between IgE and its high affinity receptor Fc fragment expressed on mast cell, is a critical step in the development of an allergic reaction and is therefore a major focus of attention of the development of strategies to intervene at the molecular level to control allergy.

Peptide 96/199 is related to the sequence of IgE Fc fragment. It has exhibited dose dependent (0.5-5.0 mg/kg po) anti passive cutaneous anaphylaxis (PCA) activity (43 to 82%) in rats. It has also showed dose dependent (1.0 to 5.0 mg *po*) mast cell stabilising both in normal (55 to 84%) and sensitised (47 to 72%) mast cells.

Further it has been studied in Schultz Dale phenomenon (1 to 3 µg/mL showed 60 to 90% blockade) and arosol tests in senstised g. pigs (2.5 to 5.0 mg/kg po) where it has shown 55 to 60.5% protection. It is devoid of any other pharmacological activity. This activity of peptide 96/199 was comarable with disodium cromoglycate (50 mg/kg ip) which is a standard antiallergic drug effective by inhalation.

Nasal formulation of peptide 96/199 has also been developed and evaluated for anti PCA and mast cell stabilising activity in rats by nasal route. The nasal formulation has shown similar activity as by oral route.

**Table I: Effect of 96-1999 and DSCG on passive cutaneous anaphylaxis (PCA) test in rats.**

| **Compound** | **Dose (mg/kg)** | **% Anti-PCA activity** |
|---|---|---|
| 96-199 | 0.5 (PO) | 43 |
| ,, | 1.0 (PO) | 48 |
| ,, | 2.5 (PO) | 74 |
| ,, | 50.0 (PO) | 82 |
| DSCG | 50.0 (IP) | 77 |

**Table II: Effect of 96/199 and DSCG on mast cell degranulation by egg albumin or comp 48/80 in sensitised mast cells respectively**

| **Compound** | **Dose (mg/kg) x days** | **Sensitised mast cells (Immunological: Egg albumin)** | **Normal mast cells (Noninnunological: Comp. 48/80)** |
|---|---|---|---|
| | | % Protection | |
| 96/199 | 0.5 pro (0.1 x 5 d) | 34 | 39 |
| ,, | 1.0 pro (0.2 x 5 d) | 47 | 55 |
| ,, | 2.5 pro (0.5 x 5 d) | 66 | 77 |
| ,, | 5.0 pro (1.0 x 5 d) | 72 | 84 |
| DSCG | 50.0 pro (10.0 x 5 d) | 71 | 72 |

**Table III: Inhibition of antigen (egg albumin) induced contraction of sensitised guinea pig ileum (Schultz-Dole Phenomenon) by 96-199 and DSCG**

| **Concentration** | **% Protection to ileum contraction induced by egg albumin with** | |
|---|---|---|
| 1.0 | 60 | **No block** |
| 2.0 | 78 | 18 |
| 3.0 | 92 | 59 |

**Table IV: Aerosol test in normal and sensitised guinea pigs**

| Compound | Dose (mg/kg) | % Protection | |
|---|---|---|---|
| | | Histamine | Egg-albumin |
| 96-199 | 2.5 (po) | Nil | 55 |
| ,, | 5.0 (po) | 10 | 61 |
| DSCG | 50 (ip) | 17 | 64 |

**Biological activity of formulations:** Formulations as prepared in examples 9 to 11 were tested in rat at 5 mg/kg by nasal route for anti PCA activity. Two formulations showed 63% (example 10) and 80% (example 11) activity, which was comparable with that of disodium cromoglycate (DSCG) at 50 mg/kg by nasal route, which showed 81% activity while the other formulation (example 1) showed no significant activity.

The study was also done in rats at 1 mg/kg by nasal route daily for five days. There was 61% (example 10) and 66% (example 11) protection of mast cells, while the other formulation (example 9) showed no significant activity:
**Other Pharmacological Studies:** Peptide 96-199 was further evaluated for its cardiovascular, Central Nervous System (CNS), anti-inflammatory, diuretic activities. No significant effects were observed up to a dose of 10 mg/kg.

The following examples are provided by the way of illustration of the present invention and should in no way be construed as a limitation thereof including the linker amide bond between the amino acids which may CH₂NH, CH₂S etc.

### Example 1

To a suspension of 4-alkoxybenzylalcohol resin (0.25 gm, 0.14 m.mol) in dry methylene chloride (6 ml), N-α-9-fluorenyl methoxycarbonyl-L-Lysine(t-butyloxycarbonyl) (0.2526 gm, 0.56 m.mol), (Boc)₂O (0.1177 gm, 0.56 m.mol), pyridine (0.435 ml, 0.56 m.mol) and dimethylamino pyridine (0.0049 gm, 0.04 m.mol) were added and the mixture stirred slowly at 0°C under anhydrous condition for 18 hr. The Lys content of the N-α-9-fluorenyl methoxy carbonyl-L-Lysyl-resin estimated to be 0.40 m.mol/g of resin. The N-α-9-fluorenyl methoxy carbonyl-L-Lysyl resin was subjected to basic cleavage and subsequently coupling in a G-3 sintered funnel using N₂ agitation in a following manner: washing with dimethylformamide (3x2 min); deblocking with 20% piperidine in dimethylformamide (1x15 min); washing with dimethylformamide, isopropanol and dimethylformamide (3x2 min). Coupling with N-α-9-fluorenylmethoxycarbonyl-sarcosine (0.13gm, 0.42m.mol), 1-hydroxybenzotrazole (0.056gm, 0.42m.mol), diisopropylcarbodiimide (0.065 ml, 0.42 m.mol) and dimethylformamide (5 ml) (1x120 min); dimethylformamide (3x1 min); isopropanol (3x1 min) and dichloromethane (3x1 min). All washing and reactions were carried out with 5ml portion of solvent. The resulting protected dipeptide 9-fluoronyl methoxy carbonyl-sarcosyl-L-lysyl resin was then dried in a vacuum dessicator. The protected dipeptide resin was subjected to deblocking and then coupling with N-α-9-fluorenyl-L-aspartic acid (t-butyl) in a following manner; deblocking with 20% piperidine/dimethylformamide (5ml; 1x15min); washing with dimethylformamide (3x2min); isopropanol (3x2min); dimethylformamide (3x2min); coupling with Fmoc-L-Asp(But)-OH (0.172 gm, 0.42 m.mol), 1-hydroxybenzotriazole (0.056 gm, 0.42 m.mol) and N,N'-diisopropylcarbodiimide (0.065 ml, 0.42m.mol) (1x120 min); washing with dimethylformamide (8 mlx3x2 min), isopropanol (8 ml x 3 x 2 min); and dichloromethane (8 ml x 3 x 2 min). All washing and reactions were carried out with 5 ml portion of solvent. The resulting tripeptide N-α-9-fluorenyl methoxy carbonyl-L-aspartyl (t-butyl)-sarcosyl-L-lysyl (t-butyloxy carbonyl) Wang's resin was then dried in dessicator. The protected tripeptide resin was subjected to deblocking and then coupling with N-α-9-fluorenyl methoxycarbonyl-glycine (0.125 gm, 0.42 m.mol) exactly in a manner described earlier. The resulting tetrapeptide N-α-9-fluorenyl methoxy carbonyl-glycyl-L-aspartyl (t-butyl)-sarcosyl-L-lysysl (t-butyloxy carbonyl) Wang's resin was then dried in vacuum dessicator. The protected tetrapeptide was subjected to deblocking and then coupling with N-α-9-fluorenyl methoxy carbonyl-glycine (0.125 gm, 0.42 m.mol) exactly in a manner described earlier. The resulting pentapeptide N-α-9-fluorenyl methoxy carbonyl- glycyl-glycyl- L-aspartyl(t-butyl)-sarcosyl-L-lysyl(t-butyloxy carbonyl) Wang's resin was then dried in vacuum dessicator. The protected penta peptide was subjected to deblocking and then coupling with N-α-t-butyloxy carbonyl-L-alanine (0.0765 gm, 0.42 m.mol) exactly in a manner described earlier. The resulting hexapeptide N-α-t-butyloxy carbonyl-L-alanyl-glycyl-glycyl- L-aspartyl (t-butyl)-sarcosyl-L-lysyl (t-butyloxy carbonyl) resin was then dried *in vaccuo.* The protected hexapeptide resin was treated with trifluoroacetic acid (9.50 ml), anisole (0.25 ml) and water (0.25 ml) with very slowly N₂ agitation for 2 hrs, in G-3 sintered reaction vessel. After this resin was filtered and washed thoroughly with TFA/DCM mixture filterate was concentrated *in vacuuo* and precipitated with diethyl ether (50 ml). The precipitate was kept at 0°C for 30 min and then at room temperature for 30 min. The precipitate was filtered and dried *in vacuuo*. The product L-Alanyl-Glycyl- Glycyl-L-Aspartyl-Sarcosyl-L-Lysyl of the formula **1** was then dried in vacuum dessicator. The yield of pure compound is 124 mg, FAB MS: 518 (M+H)

### Example 2

To a suspension of 4-alkoxybenzylalcohol resin (0.25 gm) in dry dimethyl formamide (6 ml), N-α-9-fluoronyl methoxycarbonyl-L-lysine(t-butyloxycarbonyl) (0.25 gm, 0.56 mmol), (Boc)₂O (0.1177 gm, 0.56 mmol), pyridine (0.435 ml, 0.56 mmol) and dimethylamino pyridine (0.0049 gm, 0.040 mmol) were added and the mixture stirred slowly at 0°C under anhydrous condition for 18 hr. The Lys content of the N-α-9-fluorenyl methoxy carbonyl-L-lysyl resin of estimated to be 0.40 m.mol/g of resin. The N-α-9-fluorenylmethoxy carbonyl-L-lysyl resin of formula 1 was subjected to basic cleavage and subsequently coupling in a G-3 sintered funnel using N₂ agitation in a following manner: washing with dimethylformamide(3x2 min); deblocking with 20% piperidine in dimethylformamide (1x15 min); washing with dimethylformamide, isopropanol and dimethylformamide (3x2 min). Coupling with N-α-9-fluorenylmethoxycarbonyl-glycine (0.125 gm, 0.42 m.mol), 1-hydroxybenzotrazole (0.183 gm, 1.20 m.mol), dicyclohexylcarbodiimide (0.247 gm, 1.2 m.mol) and dimethylformamide (5 ml) (1x120 min); dimethylformamide (3x1 min); isopropanol (3x1 min) and dichloromethane (3x1 min). All washing and reactions were carried out with 5 ml portion of solvent. The resulting protected dipeptide 9-fluoronyl methoxy carbonyl-glycyl-L-lysyl resin was then dried in a vacuum dessicator. The protected dipeptide resin was subjected to deblocking and then coupling with N-α-9-fluorenylmethyloxycarbonyl-L-aspartic acid (t-butyl)(0.493 gm, 1.20 m.mol) in the following manner: deblocking with 20% piperidine/dimethyl-formamide (5 ml x I x 15 min); washing with dimethyl formamide (3 x 2 min); isopropanol (3x2 min); dimethyl formamide (3x2 min); coupling with Fmoc-L-Asp(But)-OH (0.493 g, 1.20 m.mol), 1-hydroxy benzotriazole (0.183 gm, 1.20 m.mol) and N,N'-dicyclohexylcarbodiimide (0.247 gm , 1.20 m.mol) (1x120 min); washing with dimethylformamide (8 ml x 3 x 2 min), isopropanol (8 ml x 3 x 2 min); and dichloromethane (8 ml x 3 x 2 min). All washings and reactions were carried out with 5 mL portion of solvent. The resulting tripeptide N-α-9-fluorenyl methoxy carbonyl-L-aspartyl (t-butyl)-glycyl-L-lysyl (t-butyloxy carbonyl) Wang's resin was then dried in dessicator. The protected tripeptide resin was subjected to deblocking and then coupling with N-α-9-fluorenyl methoxycarbonyl-glycine (0.356gm, 1.20mmol) exactly in a manner described earlier. The resulting tetrapeptide N-α-9-fluorenyl methoxycarbonyl-glycyl-L-aspartyl (t-butyl)-glycyl-L-lysyl (t-butyloxycarbonyl) Wang's resin was then dried in vacuum dessicator. The protected tetrapeptide was subjected to deblocking and then coupling with N-α-9-fluorenyl methoxy carbonyl-Sarcosine (0.13 gm, 0.42 mmol) exactly in a manner described earlier. The resulting pentapeptide N-α-9-fluorenyl methoxycarbonylsarcosyl-glycyl- L-aspartyl(t-butyl)- glycyl- L-lysyl(t-butyloxy carbonyl) Wang's resin was then dried in vacuum dessicator. The protected pentapeptide was subjected to deblocking and then coupling with N-α-t-butyloxy carbonyl-L-alanine (0.227 gm, 1.20 m.mol) exactly in a manner described earlier. The resulting hexapeptide N-α-t-butyloxy carbonyl-L-alanyl-sarcosyl-glycyl- L-aspartyl (t-butyl)-glycyl-L-lysyl (t-butyloxycarbonyl) resin was then dried *in vacuo*. The protected hexapeptide resin (0.2500 gm) was treated with trifluoroacetic acid (9.50 ml), anisole (0.25 ml) and water (0.25 ml) with very slowly N₂ agitation for 2 hrs, in G-3 sintered reaction vessel. After this resin was filtered and washed thoroughly with TFA/DCM mixture filtrate was concentrated *in vaccuo* and precipitated with diethyl ether (50 ml). The precipitate was kept at O°C for 30 min and then at room temp. for 30 min. The precipitate was filter & dried *in vacuo*. The product L-alanyl-sarcosyl-glycyl-L-aspartyl-glycyl-L-lysyl of the formula **1** was then dried in vacuum dessicator. The yield of pure compound is 115 mg, FAB MS: 518 (M+H)

### Example 3

To a suspension of 4-alkoxybenzylalcohol resin (0.2500 gm) in a mixture of dry dimethyl formamide -dry methylene chloride (3 + 3 ml), N-α-9-fluoronylmethoxycarbony 1-L-lysine (t-butyloxycarbonyl) (0.2526 gm, 0.56 mmol), (Boc)₂O (0.1177 gm, 0.56 mmol), pyridine (0.435 ml, 0.56 mmol) and dimethylamino pyridine (0.0049 gm, 0.040 mmol) were added and the mixture stirred slowly at 20°C under anhydrous condition for 18 hr. Lys content of the N-α-9-fluorenyl methoxy carbonyl-L-lysyl resin estimated to be 0.38 m.mol/g of resin. The N-α-9-fluorenylmethoxy carbonyl-L-lysyl resin was subjected to basic cleavage and subsequently coupling in a G-3 sintered funnel using N₂ agitation in a following manner: washing with dimethyl formamide (3x2 min); deblocking with 20% piperidine in dimethyl formamide (1x15 min); washing with dimethylformamide, isopropanol and dimethylformamide (3x2 min). Coupling with N-α-9-fluorenylmethoxycarbonyl-glycine (0.356 gm, 1.20 m.mol), benzotriazole-1-yl-oxytripyrrolidinephosphonium hexafluorophophate (0 .541 gm, 1.04 m.mol) and diisopropylethylamine (0.181 mL, 1.04 m.mol) in dimethylformamide (5 ml) (1x120 min); dimethylformamide (3x1 min); isopropanol (3x1 min) and dichloromethane (3x1 min). All washing and reactions were carried out with 5 ml portion of solvent. The resulting protected dipeptide 9-fluorenyl methoxy carbonyl-glycyl-L-lysyl resin of formula **4** was then dried in a vacuum dessicator. The protected dipeptide resin of formula **4** was subjected to deblocking and then coupling with N-α-9-fluorenylmethyloxycarbonyl-L-aspartic acid (t-butyl) (0.427 gm, 1.04m.mol) in the following manner; deblocking with 20% piperidine/dimethylformamide (5mlx1x15min); washing with dimethyl formamide (3x2min); isopropanol (3x2min); dimethylformamide (3x2min); coupling with Fmoc-L-Asp(But)-OH (0.427 g, 1.04 m.mol), PyBOP(0.541 gm, 1.04 m.mol) and diisopropylethylamine (0.181 ml, 1.04 m.mol) in dimethylformamide (8 ml x 3 x 2 min), isopropanol (8 ml x 3 x 2 min); and dichloro methane (8 ml x 3 x 2 min). All washing and reactions were carried out with 5 ml portion of solvent. The resulting tripeptide N-α-9-fluorenyl methoxy carbonyl-L-aspartyl (t-butyl)-glycyl-L-lysyl (t-butyloxy carbonyl) Wang's resin was then dried in dessicator. The protected tripeptide resin form was subjected to deblocking and then coupling with N-α-9-fluorenyl methoxy carbonyl-sarcosine (0.13 gm, 0.42 m.mol) exactly in a manner described for **6.** The resulting tetrapeptide N-α-9-fluorenyl methoxy carbonyl-sarcosyl-L-aspartyl (t-butyl)- glycyl 1-L-lysyl (t-butyloxy carbonyl) Wang's resin was then dried in vacuum dessicator. The protected tetrapeptide was subjected to deblocking and then coupling with N-α-9-fluorenyl methoxy carbonyl- sarcosine (0.13 gm, 0.42 m.mol) exactly in a manner described for **6.** Resulting pentapeptide N-α-9-fluorenyl methoxy carbonyl-sarcosyl-sarcosyl- L-aspartyl(t-butyl) - glycyl -L-lysyl(t-butyloxy carbonyl) Wang's resin was then dried in vacuum dessicator. Protected pentapeptide was subjected to deblocking and then coupling with N-α-t-butyloxy carbonyl-L-alanine (0.026 gm, 0.42m.mol) exactly in the manner described above. Resulting hexapeptide N-α-t-butyloxy carbonyl-L-alanyl-sarcosyl-sarcosyl - L-aspartyl (t-butyl)-glycyl-L-lysyl (t-butyloxy carbonyl) resin was then dried *in vaccuo.* The protected hexapeptide resin (0.25gm) was treated with trifluoro acetic acid (9.50ml), anisole (0.25ml) and water (0.25 ml) with very slowly N₂ agitation for 2 hrs, in G-3 sintered reaction vessel. After this resin was filtered and washed thoroughly with TFA/DCM mixture filterate was concentrated *in vaccuo* and precipitated with diethyl ether (50 ml). The precipitate was kept at 0°C for 30 min and then at room temp. for 30 min. The precipitate was filter & dried *in vacuo.* The product L-alanyl- sarcosyl - sarcosyl -L-aspartyl- glycyl -L-lysyl of the formula 1 was then dried in vacuum dessicator. The yield of pure compound is 120 mg, FAB MS: 532 (M+H)

### Example4

The coupling of N- α-9-fluoronyl methoxycarbonyl-L-lysine(t-butyloxycarbonyl) to Rink Amide resin was carried out in two steps: 1) Rink amide resin (0.25 gm) was treated with 20% piperidine/DMF slution for 25 min at rt. After this the resin was succeesivley washed with DMF (3 x 2 min), iPrOH (3 x 2 min) and DMF (3 x 2 min). It was then treated with N-α-9-fluoronyl methoxycarbonyl-L-lysine(t-butyloxycarbonyl) (0.22 gm, 0.48 m.mol), HOBt (0.073 gm, 0.48 m.mol), and DIC (0.063 ml, 0.48 m.mol) for 12 hr. The resin was drained and successively washed with dimethylformamide (3x1 min); isopropanol (3x1 min) and dichloromethane (3x1 min) to get Lysyl derivative. The completion of reaction was monitored by negative Kaiser test. The N-α-9-fluorenylmethoxy carbonyl-L-lysyl resin was subjected to basic cleavage and subsequently coupling in a G-3 sintered funnel using N₂ agitation in a following manner: washing with dimethylformamide (3x2 min); deblocking with 20% piperidine in dimethylformamide (1x15 min); washing with dimethylformamide, isopropanol and dimethylformamide (3x2 min). Coupling with N-α-9-fluorenylmethoxycarbonyl-glycine (0.125 gm, 0.42 m.mol), 1-hydroxybenzotrazole (0.183 gm, 1.20 m.mol), dicyclohexylcarbodiimide (0.247 gm, 1.2 m.mol) in dimethylformamide (5 ml) (1x120 min) and washing with dimethylformamide (3x1 min); isopropanol (3x1 min) and dichloromethane (3x1 min). All washings and reactions were carried out with 5 mL portion of solvent. The resulting protected dipeptide 9-fluoronyl methoxy carbonyl-glycyl-L-lysyl resin was then dried in a vacuum dessicator. The protected dipeptide resin was subjected to deblocking and then coupling with N-α-9-fluorenylmethyloxycarbonyl-L-aspartic acid (t-butyl)(0.493 gm, 1.20 m.mol) in a following manner; deblocking with 20% piperidine/dimethylformamide (5 ml x 1 x 15 min); washing with dimethylformamide (3 x 2 min); isopropanol (3x2 min); dimethylformamide (3x2 min); coupling with Fmoc-L-Asp(But)-OH (0.493 g, 1.20 m.mol), 1-hydroxybenzotriazole (0.183 gm, 1.20 m.mol) and N,N'-dicyclohexylcarbodiimide (0.247 gm , 1.20 m.mol) (1x120 min); washing with dimethylformamide (8 ml x 3 x 2 min), isopropanol (8 ml x 3 x 2 min); and dichloromethane (8 ml x 3 x 2 min). All washings and reactions were carried out with 5 mL portion of solvent. The resulting tripeptide N-α-9-fluorenyl methoxy carbonyl-L-aspartyl (t-butyl)-glycyl-L-lysyl (t-butyloxy carbonyl) amide resin was then dried in dessicator. The protected tripeptide resin was subjected to deblocking and then coupling with N-α-9-fluorenyl methoxy carbonyl-glycine (0.356 gm, 1.20 m.mol) exactly in a manner described earlier. The resulting tetrapeptide N-α-9-fluorenyl methoxy carbonyl-glycyl-L- aspartyl (t-butyl)-glycyl-L-lysysl (t-butyloxy carbonyl) amide resin was then dried in vacuum dessicator. The protected tetrapeptide was subjected to deblocking and then coupling with N-α-9-fluorenyl methoxy carbonyl-sarcosine (0.13 gm, 0.42 m.mol) exactly in a manner described earlier. The resulting pentapeptide N-α-9-fluorenyl methoxy carbonyl- sarcosyl-glycyl- L-aspartyl(t-butyl)- glycyl-L-lysyl(t-butyloxy carbonyl) amide resin was then dried in vacuum dessicator. The protected pentapeptide was subjected to deblocking and then coupling with N-α-t-butyloxy carbonyl-N-allyl-L-alanine (0.096 gm, 0.42 m.mol) exactly in a manner described earlier. The resulting hexapeptide N-α-t-butyloxy carbonyl- N-allyl L-alanyl-sarcosyl-glycyl- L-aspartyl (t-butyl)-glycyl-L-lysyl (t-butyloxycarbonyl) resin was then dried *in vacuo*. The protected hexapeptide resin (0.2500 gm) was treated with trifluoroacetic acid (9.50 ml), anisole (0.25 ml) and water (0.25 ml) with very slowly N₂ agitation for 2 hrs, in G-3 sintered reaction vessel. After this resin was filtered and washed thoroughly with TFA/DCM mixture filtrate was concentrated *in vaccuo* and precipitated with diethyl ether (50 ml). The precipitate was kept at O°C for 30 min and then at room temp. for 30 min. The precipitate was filter & dried *in vacuo*. The product N-allyl-L-alanyl-sarcosyl-glycyl-L-aspartyl-glycyl-L-lysyl-amide of the formula 1 was then dried in vacuum dessicator. The yield of pure compound is 115 mg, FAB MS: 517 (M+H)

### Example 5

The coupling of N- α-9-fluoronyl methoxycarbonyl-L-lysine(t-butyloxycarbonyl) to Rink Amide resin was carried out in two steps: 1) Rink amide resin (0.25 gm) was treated with 20% piperidine/DMF slution for 25 min at rt. After this the resin was succeesivley washed with DMF (3 x 2 min), iPrOH (3 x 2 min) and DMF (3 x 2 min). It was then treated with N-α-9-fluoronyl methoxycarbonyl-L-lysine(t-butyloxycarbonyl) (0.22 gm, 0.48 m.mol), HOBt (0.073 gm, 0.48 m.mol), and DIC (0.063 ml, 0.48 m.mol) for 12 hr. The resin was drained and successively washed with dimethylformamide (3x1 min); isopropanol (3x1 min) and dichloromethane (3x1 min) to get Lysyl derivative. The completion of reaction was monitored by negative Kaiser test. The N-α-9-fluorenylmethoxy carbonyl-L-lysyl resin was subjected to basic cleavage and subsequently coupling in a G-3 sintered funnel using N₂ agitation in a following manner: washing with dimethyl formamide (3x2 min); deblocking with 20% piperidine in dimethyl formamide (1x15 min); washing with dimethylformamide, isopropanol and dimethylformamide (3x2 min). Coupling with N-α-9-fluorenylmethoxycarbonyl-glycine (0.356 gm, 1.20 m.mol), Benzotriazole-1-yl-oxytripyrrolidinephosphonium hexafluorophophate (0 .541 gm, 1.04 m.mol) and diisopropylethylamine (0.181 ml, 1.04 m.mol) in dimethylformamide (5 ml) (1x120 min); dimethylformamide (3x1 min); isopropanol (3x1 min) and dichloromethane (3x1 min). All washing and reactions were carried out with 5 ml portion of solvent. The resulting protected dipeptide 9-fluorenyl methoxy carbonyl-glycyl-L-lysyl resin was then dried in a vacuum dessicator. The protected dipeptide resin was subjected to deblocking and then coupling with N-α-9-fluorenylmethyloxycarbonyl-L-Aspartic acid (t-butyl) (0.427 gm, 1.04 m.mol) in a following manner; deblocking with 20% piperidine/dimethylformamide (5 ml x 1 x 15 min); washing with dimethyl formamide (3 x 2 min); isopropanol (3x2 min); dimethylformamide (3x2 min); coupling with Fmoc-L-Asp(But)-OH (0.427 g, 1.04 m.mol), PyBOP(0.541 gm, 1.04 m.mol) and diisopropylethylamine (0.181 ml, 1.04 m.mol) in dimethylformamide (8 ml x 3 x 2 min), isopropanol (8 ml x 3 x 2 min); and dichloro methane (8 ml x 3 x 2 min). All washing and reactions were carried out with 5 ml portion of solvent. The resulting tripeptide N-α-9-fluorenyl methoxy carbonyl-L-aspartyl (t-butyl)-glycyl-L-lysyl (t-butyloxy carbonyl) amide resin was then dried in dessicator. The protected tripeptide resin was subjected to deblocking and then coupling with N-α-9-fluorenyl methoxy carbonyl-Gly (0.13 gm, 0.42 m.mol) exactly in a manner described as described earlier. The resulting tetrapeptide N-α-9-fluorenyl methoxy carbonyl- glycyl-L-aspartyl (t-butyl)- glycyl-L-lysyl (t-butyloxy carbonyl)amide resin was then dried in vacuum dessicator. The protected tetrapeptide was subjected to deblocking and then coupling with N-α-9-fluorenyl methoxy carbonyl- N-allylglycyl (0.16 gm, 0.48 m.mol) exactly in a manner described earlier . The resulting pentapeptide N-α-9-fluorenyl methoxy carbonyl- allylglycyl-glycyl L-aspartyl(t-butyl) - glycyl -L-lysyl(t-butyloxy carbonyl) amide resin was then dried in vacuum dessicator. The protected penta peptide was subjected to deblocking and then coupling with N-α-t-butyloxy carbonyl-L-alanine (0.197 gm, 1.04 m.mol) exactly in a manner described earlier. The resulting hexapeptide N-α-t-butyloxy carbonyl-L-alanyl-N-allylglycyl-glycyl - L-aspartyl (t-butyl)-glycyl-L-lysyl (t-butyloxy carbonyl) resin of was then dried *in vaccuo.* The protected hexapeptide resin (0.25 gm) was treated with trifluoro acetic acid (9.50 ml), anisole (0.25 ml) and water (0.25 ml) with very slowly N₂ agitation for 2 hrs, in G-3 sintered reaction vessel. After this resin was filtered and washed thoroughly with TFA/DCM mixture filtrate was concentrated *in vacuo* and precipitated with diethyl ether (50 ml). The precipitate was kept at O°C for 30 min and then at room temperature for 30 min. The precipitate was filtered & dried *in vacuo*. The product L-alanyl- N-allylglycyl - glycyl -L-aspartyl- glycyl -L-lysyl-amide of the formula **1** was then dried in vacuum dessicator. The yield of pure compound is 120 mg, FAB MS: 543 (M+H)

### Example 6

To a suspension of 4-alkoxybenzylalcohol resin (0.2500 gm) in a mixture of dry dimethyl formamide -dry methylene chloride (3 + 3 ml), N-α-9-fluoronylmethoxycarbony 1-L-Lysine (t-butyloxycarbonyl) (0.2526 gm, 0.56 m.mol), (Boc)₂O (0.1177 gm, 0.56 m.mol), pyridine (0.435 ml, 0.56 m.mol) and dimethylamino pyridine (0.0049 gm, 0.040 m.mol) were added and the mixture stirred slowly at 20°C under anhydrous condition for 18 hr. The Lys content of the N-α-9-fluorenyl methoxy carbonyl-L-lysyl resin estimated to be 0.38 m.mol/g of resin. The N-α-9-fluorenylmethoxy carbonyl-L-lysyl resin was subjected to basic cleavage and subsequently coupling in a G-3 sintered funnel using N₂ agitation in a following manner: washing with dimethyl formamide (3x2 min); deblocking with 20% piperidine in dimethyl formamide (1x15 min); washing with dimethylformamide, isopropanol and dimethylformamide (3x2 min). Coupling with N-α-9-fluorenylmethoxycarbonyl-Glycine (0.356 gm, 1.20 m.mol), Benzotriazole-1-yl-oxytripyrrolidinephosphonium hexafluorophophate (0 .541 gm, 1.04 m.mol) and- diisopropylethylamine (0.181 ml, 1.04 m.mol) in dimethylformamide (5 ml) (1x120 min); dimethylformamide (3x1 min); isopropanol (3x1 min) and dichloromethane (3x1 min). All washing and reactions were carried out with 5 ml portion of solvent. The resulting protected dipeptide 9-fluorenyl methoxy carbonyl-glycyl-L-lysyl resin was then dried in a vacuum dessicator. The protected dipeptide resin was subjected to deblocking and then coupling with N-α-9-fluorenylmethyloxycarbonyl-L-aspartic acid (t-butyl) (0.427 gm, 1.04 m.mol) in a following manner; deblocking with 20% piperidine/dimethylformamide (5 ml x 1 x 15 min); washing with dimethyl formamide (3 x 2 min); isopropanol (3x2 min); dimethylformamide (3x2 min); coupling with Fmoc-L-Asp(But)-OH (0.427 g, 1.04 m.mol), PyBOP(0.541 gm, 1.04 m.mol) and diisopropylethylamine (0.181 mL, 1.04 m.mol) in dimethylformamide (8 ml x 3 x 2 min), isopropanol (8 ml x 3 x 2 min); and dichloro methane (8 ml x 3 x 2 min). All washing and reactions were carried out with 5 ml portion of solvent. The resulting tripeptide N-α-9-fluorenyl methoxy carbonyl-L-aspartyl (t-butyl)-glycyl-L-lysyl (t-butyloxy carbonyl) Wang's resin was then dried in dessicator. The protected tripeptide resin was subjected to deblocking and then coupling with N-α-9-fluorenyl methoxy carbonyl-N-allylglycyl (0.16 gm, 0.48 m.mol) exactly in a manner described as earlier. The resulting tetrapeptide N-α-9-fluorenyl methoxy carbonyl- N-allylglycyl -L-aspartyl (t-butyl)- glycyl 1-L-lysyl (t-butyloxy carbonyl) Wang's resin was then dried in vacuum dessicator. The protected tetrapeptide was subjected to deblocking and then coupling with N-α-9-fluorenyl methoxy carbonyl- Glycine (0.14 gm, 0.48 m.mol) exactly in a manner described for **6.** The resulting pentapeptide N-α-9-fluorenyl methoxy carbonyl- glycyl-N-allyglycyl- L-aspartyl(t-butyl) - glycyl -L-lysyl(t-butyloxy carbonyl) Wang's resin was then dried in vacuum dessicator. The protected pentapeptide was subjected to deblocking and then coupling with N-α-t-butyloxy carbonyl-L-alanine (0.026 gm, 0.42m.mol) exactly in the manner described earlier. The resulting hexapeptide N-α-t-butyloxy carbonyl-L-alanyl- glycyl-N-allyglycyl - L-aspartyl (t-butyl)-glycyl-L-lysyl (t-butyloxy carbonyl) resin was then dried *in vaccuo.* The protected hexapeptide resin (0.25 gm) was treated with trifluoro acetic acid (9.50 ml), anisole (0.25ml) and water (0.25 ml) with very slowly N₂ agitation for 2 hrs, in G-3 sintered reaction vessel. After this resin was filtered and washed thoroughly with TFA/DCM mixture filterate was concentrated *in vaccuo* and precipitated with diethyl ether (50 ml). The precipitate was kept at 0°C for 30 min and then at room temp. for 30 min. The precipitate was filter & dried *in vacuo*. The product L-alanyl-glycyl-N-allyglycyl -L-aspartyl- glycyl -L-lysyl of the formula **1** was then dried in vacuum dessicator. The yield of pure compound is 120 mg, FAB MS: 544 (M+H)

### Example 7

To a suspension of 4-alkoxybenzylalcohol resin (0.2500 gm) in a mixture of dry dimethyl formamide -dry methylene chloride (3 + 3 ml), N-α-9-fluoronylmethoxycarbony 1-L-lysine (t-butyloxycarbonyl) (0.2526 gm, 0.56 m.mol), (Boc)₂O (0.1177 gm, 0.56 m.mol), pyridine (0.435 ml, 0.56 m.mol) and dimethylamino pyridine (0.0049 gm, 0.040 m.mol) were added and the mixture stirred slowly at 20°C under anhydrous condition for 18 hr. The Lys content of the N-α-9-fluorenyl methoxy carbonyl-L-Lysyl resin of formula 2 estimated to be 0.38 m.mol/g of resin. The N-α-9-fluorenylmethoxy carbonyl-L-lysyl resin was subjected to basic cleavage and subsequently coupling in a G-3 sintered funnel using N₂ agitation in a following manner: washing with dimethyl formamide (3x2 min); deblocking with 20% piperidine in dimethyl formamide (1x15 min); washing with dimethylformamide, isopropanol and dimethylformamide (3x2 min). Coupling with N-α-9-fluorenyl methoxy carbonyl-N-allylglycyl (0.16 gm, 0.48 m.mol), Benzotriazole-1-yl-oxytripyrrolidinephosphonium hexafluorophophate (0 .541 gm, 1.04 m.mol) and diisopropylethylamine (0.181 mL, 1.04 m.mol) in dimethylformamide (5 ml) (1x120 min); dimethylformamide (3x1 min); isopropanol (3x1 min) and dichloromethane (3x1 min). All washing and reactions were carried out with 5 mL portion of solvent. The resulting protected dipeptide 9-fluorenyl methoxy carbonyl-N-allylglycyl-L-lysyl resin was then dried in a vacuum dessicator. The protected dipeptide resin of formula **4** was subjected to deblocking and then coupling with N-α-9-fluorenylmethyloxycarbonyl-L-aspartic acid (t-butyl) (0.427 gm, 1.04 m.mol) in a following manner; deblocking with 20% piperidine/dimethylformamide (5 ml x 1 x 15 min); washing with dimethyl formamide (3 x 2 min); isopropanol (3x2 min); dimethylformamide (3x2 min); coupling with Fmoc-L-Asp(But)-OH (0.427 g, 1.04 m.mol), PyBOP(0.541 gm, 1.04 m.mol) and diisopropylethylamine (0.181 mL, 1.04 m.mol) in dimethylformamide (8 ml x 3 x 2 min), isopropanol (8 ml x 3 x 2 min); and dichloro methane (8 ml x 3 x 2 min). All washing and reactions were carried out with 5 ml portion of solvent. The resulting tripeptide N-α-9-fluorenyl methoxy carbonyl-L-aspartyl (t-butyl)-N-allylglycyl-L-lysyl (t-butyloxy carbonyl) Wang's resin was then dried in dessicator. The protected tripeptide resin was subjected to deblocking and then coupling with N-α-9-fluorenyl methoxy carbonyl-glycine (0.14 gm, 0.48 m.mol) exactly in a manner as described earlier. The resulting tetrapeptide N-α-9-fluorenyl methoxy carbonyl-glycyl -L-aspartyl (t-butyl)- N-allylglycyl -L-lysyl (t-butyloxy carbonyl) Wang's resin was then dried in vacuum dessicator. The protected tetrapeptide was subjected to deblocking and then coupling with N-α-9-fluorenyl methoxy carbonyl- glycine (0.14 gm, 0.48 m.mol) exactly in a manner as described earlier. The resulting pentapeptide N-α-9-fluorenyl methoxy carbonyl- glycyl-glycyl- L-lspartyl(t-butyl) - N-allylglycyl -L-lysyl(t-butyloxy carbonyl) Wang's resin was then dried in vacuum dessicator. The protected pentapeptide was subjected to deblocking and then coupling with N-α-t-butyloxy carbonyl-L-alanine (0.026 gm, 0.42 m.mol) exactly in a manner as described earlier. The resulting hexapeptide N-α-t-butyloxy carbonyl-L-alanyl-glycyl- glycyl - L-aspartyl (t-butyl)- N-allyl glycyl-L-lysyl (t-butyloxy carbonyl) resin was then dried *in vaccuo.* The protected hexapeptide resin (0.25 gm) was treated with trifluoro acetic acid (9.50 ml), anisole (0.25 ml) and water (0.25 ml) with very slowly N₂ agitation for 2 hrs, in G-3 sintered reaction vessel. After this resin was filtered and washed thoroughly with TFA/DCM mixture filterate was concentrated *in vaccuo* and precipitated with diethyl ether (50 ml). The precipitate was kept at O°C for 30 min and then at room temp. for 30 min. The precipitate was filter & dried *in vacuo*. The product L-alanyl- glycyl- glycyl -L-aspartyl-N-allyl glycyl -L-lysyl of the formula **1** was then dried in vacuum dessicator. The yield of pure compound is 120 mg, FAB MS: 544 (M+H)

### Example 8

**ZAlaGlyOEt :** A solution of ZAla (35.234 g; 0.158 m), hydroxybenzo-triazole (24.2 g; 0.158 m) and DCC (32.55 g; 0.158m) in dichloro methane (300 ml) and DMF (100 ml) was stirred at 0C for 1 hr, (during this time dicyclohexylurea (DCU) started separating an indication of activation. The precooled mixture of glycine ethylester hydrochloride (24.00 g ; 0.173 mol) and triethylamine (24.0 ml; 0.173 mol) in dichloromethane (150 ml) was added to the reaction vessel, stirring was continued for 6 hours at 0°C then at room temperature for 15 - 16 hours. The reaction vessel was cooled to -5°C to -10 °C for 2 hours and separated dicyclo hexyl urea (DCU) was filtered off with suction and washed with cold dichloromethane 2 times. The filtrate was subjected for distillation of solvent at rotavapour (below 40°C). The residue was poured into water (500 ml) and stirred at room temperature (30°C) for 1 hour and extracted with ethyl acetate (300 ml, 200 ml), organic layer was washed with 5% sodium bicarbonate solution 2x250 ml; water 2x200 ml ; N HCl 2x200 ml; water 2x250 ml. Organic layer was separated and TLC (10% MeOH : chloroform) was checked and dried over anhydrous sodium sulphate (150 g) for 3 hours , sodium sulphate was removed by filtration , solvent was removed from mother liquor by distillation on rotavapour (below 40°C). Residue was recrystallized from ethylacetate : Hexane. Yield 39 g (80%); m.p. 101°C. [Lit 99-100°C Bull Chem Soc Japan 46 (1973)]; HPLC 98%.

**ZAla-GlyOH** : ZAla-Gly OEt (38.5; 0.125 m) was dissolved in methanol (200 ml) and added 2 N sodiumhydroxide (62.5 ml), the reaction mixture was stirred at room temperature (30 °C) for 3 hours, methanol was distilled off on rotavapour (below 40°C) residue was dissolved in water (100 ml) and 1 N HCl (110 ml) was added slowly to it the turbidity appears and oily mass separates out, extracted with ethyl acetate (500 ml). The organic layer was separated and washed thoroughly with water till neutral (4 x 200 ml H2O) and dried over anhydrous sodium sulphate (150 g). Solvent distilled off on rota vapour (below 40°C), residue recrystallized from EtOAc :Hexane. Yield 26 g (75%) . m.p. 131°C (Lit m.p.132 °C) Int.J.Peptide & Protein Res 4 177(1972).

**Z-Ala-Gly-Gly-OEt :** A mixture of ZAla Gly OH (25.2 g ;0.09 m) and hydroxy benzotrizole (13.8; 0.09 m) was dissolved in dry THF (250 ml) and stirred at 0°C. A solution of dicyclohexyl carbodiimide (18.54 g, 0.09m) in dichloromethane (75 ml) was added to reaction mixture in one lot and stirring was continued for 1.5 hours at 0°C. During this time cyclo hexyl urea (DCU) started separating an indication of activation. The precooled mixture of glycine ethyl ester hydrochloride (13.8 g; .099 m) and triethyl amine (13.8 ml; 0.099m) in dichloromethane (100 ml) was added to reaction mixture and stirring was continued for 6 hours at 0°C, then at room temperature for 30 hours. The reaction mixture was cooled to - 5°C to -10°C for 2 hours and separated dicyclohexyl urea (DCU) was filtered off with suction and washed with dichloromethane 2 times. Solvent from filtrate was distilled off on rotavapour (below 40 °C). Residue was poured into water (400 ml) and stirred at room temperature (30°C) for an hour and extracted with dichloromethane (500 ml). The organic layer was separated and washed with 5% NaHCO₃ solution 2 x 200 ml; water 2 x 150 ml; N HCl 2 x 200 ml and finally with water 2 x 150 ml and dried over anhydrous sodium sulphate (150 g) for three hours. Sodium sulphate was removed by filteration and solvent was distilled off on rotavapour (below 40 °C). Residue was recrystallized from MeOH / EtOH. Yield 28 g (85%) m.p. 135 °C [Lit 133-34 °C Tetrahedron 29 1487 (1973). TLC (10% Methanol : CHCl₃).Rf. 0.65

**Z-Ala-Gly-Gly-OH :** ZAla GlyOEt (18.5 g ; 0.05 m) was dissolved in methanol (300 ml) and stirred at 10 °C, N NaOH (50.7 ml) was added to reaction mixture at the rate of 10 ml / 5 min., initially there was some turbidity which went off after the addition of sodium hydroxide solution. Stirring was continued for 3 hours after the addition. Solvent was distilled off on rotavapor (below 40 °C) and residue extracted with ethyl acetate to remove starting material if any. Residue was dissolved in water (50 ml) and acidified with 2 N HCl (15 ml) under cooling , cooling was continued for 2 hours, separated solid was filtered and thoroughly washed with water and dried till constant weight. Yield 9.77 g (58%). TLC [CHCl₃ : MeOH : AcOH (90:8:2)] Rf. 0.55.

**Lys (2Cl- Z) benzyl ester hydrochloride :** BOC Lys (2 CL - Z) benzyl ester (38 g ; 0.075 m) was taken in TFA : DCM : : 1:1 (75 ml) and kept at room temperature (30°C) for 30 min. with occasional shaking. Solvents and excess of TFA were distilled off on rotavapour (below 40 °C) initially on water vacuum and then on high vacuum. Now dry THF (30 ml) was added to the residue and distilled off on rotavapour (below 40°C) first on water vacuum and finally on high vacuum. This process of addition of THF and removal of the solvent is repeated two times (this operation removes the traces of trifluoroacetic acid from the residue). It was cooled in ice-salt bath for 30 mts and 12% HCl -THF (50 ml) was added to it. The cooling was continued for 30 min during this time solid started separating. The excess of HCl-THF was removed on rotavapour (below 40 °C) initially on water vacuum and finally on high vacuum. Residue was cooled in ice bath and triturated with dry ether (200 ml) precipitated hydrochloride was filtered with suction and thoroughly washed with ether, dried in vacuum dessicator over phosphoruspentaoxide (P₂O₅) for 8 hrs. Yield 28 g (85%).

**BOC Sar Lys (2Cl -Z) Bzl :** BOC-Sarcosine (13.04 g; 0.069 m) and hydroxybenzotriazole (10.55 g; 0.069 m) were dissolved in tetrahydrofuran (150 ml) by stirring at 0°C, then dicyclo hexylcarbodiamide (14.2 g; 0.069 m) in dichloromethane (50 ml) was added to reaction mixture and stirring was continued at O °C for 30 mts (during this time DCU started separating an indication of activation. Then a mixture of Lys (2Cl- Z)Bzl Hydrochloride (30.46g; 0.069 m) and triethyl amine (9.6 ml; 0.069 m) in dichloromethane (100 ml) was added to reaction mixture in lots and stirring was continued at 0°C for 6 hours and room temperature (30°C) for 24 hours. Now the reaction vessel was cooled to -5°C to - 10°C for one hour and separated dicyclohexyl urea (DCU) was filtered off with suction and washed with cold dichloro-methane. Solvent from filtrate was distilled off on rotavapour (below 40°C) first on water vacuum and finally on high vacuum. Residue was taken up in water (500 ml) and extracted with ethylacetate (500 ml). Organic layer was separated and washed with water 2 x100 ml ; 5% NaHCO₃ solution 2 x 100 ml; water 2 x 100 ml; 10% citric acid 2 x 100 ml; water 2 x 100 ml and dried over anhydrous sodium sulphate. Salt was filtered off and solvent was distilled off on rotavapour (below 40 °C), residue was dried on high vacuum and finally over P₂O₅ in a vacuum desicator. Yield oil 39 g. (98%) TLC (10% Methanol : Chloroform) Rf 0.75; FAB Mass 576 , 476; HPLC: 99%.

**Sar-Lys (2Cl-Z)-OBzl. HCl :** It was prepared following the procedure out lined at No. 5 using BOC Sar Lys (2Cl - Z) Bzl (39 g; 0.068 m), TFA : DCM: : 1:1 (68 ml) HCl - THF (12% ; 45 ml). Yield 32.2 g (90%) . HPLC: 100% ; FAB Mass 476 .

**BOC-Asp-(BzI)-Sar-Lys (2Cl-Z)-OBzl :** A solution of BOC Asp(Bzl) OH (10.66 g ;0.033 m) in dichloromethane (50 ml) and hydroxybenzo-triazole (5.1 g ; 0.033 m) in dry tetrahydrofuran (50 ml) was stirred at 0°C for 10 minutes, than dicyclohexyl carbodiimide (6.80 g ; 0.033 m) in dichloromethane (20 ml) was added in one lot and stirring at 0 °C was continued for 30 minutes (during this period dicyclohexyl urea started separating an indication of activation). Now a precooled mixture of Sar Lys(2Cl - Z)Bzl hydrochloride (15.90 g ; 0.031 m), triethylamine (4.3 ml ; 0.031 m) and dichloromethane (50 ml) was added to the reaction mixture in lots. The stirring at 0°C was continued for six hours and at room temperature for 24 hours. The reaction vessel was cooled to -5 °C for two hours, separated dicyclohexyl urea was filtered off by suction and washed with cold dichloromethane. Solvent was distilled off from fitrate on rotavapour (below 40 °C). Residue was taken up in water (500 ml) and extracted with ethylacetate (400 ml). Organic layer was separated and washed with 5 % NaHCO₃ solution 120ml x 2 ; water 120ml x 2 ; 10 % citric acid 120 ml x 2 and finally with water 120 ml x 2 , dried over anhydrous sodium sulphate (100 g). After removal of salt by fiteration, solvent was ditilled off on rotavapour (below 40 °C) initially on water vacuum and finally under high vacuum. The residue was dried over phodphorus pentaoxide (P₂O₅) in a vacuum desicator for 8 hours. Yield 23.80 g (Oil ; 98 %). TLC (10 % Methanol : Chloroform); Rf: 0.7 ; HPLC : 99 % ; FAB Mass: 781 , 681.

**Asp (Bzl) Sar Lys (2Cl - Z)Bzl . Hydrochloride :** This compound was prepared following the procedure outlined at No. 5 using BOC Asp Sar Lys (2Cl -Z) Bzl (23.80 g ; 0.03 m)TFA:DCM :: 1 : 1 (30 ml) and 12 % HCL -THF (20ml). The product obtained as an oil was used in the next step with out further purification . FAB Mass : 682.

**Z-Ala-Gly-Gly-Asp(Bzl)-Sar-Lys(2Cl - Z)- OBzl** (3 + 3 coupling) : A mixture of Z Ala Gly Gly OH (9.5 g ; 0.028 m), hydroxybenzotrazole (4.29 g ; 0.028 m) and dicyclohexylcabodiimide (5.8 g ; 0.028 m) was taken in dry DMF (100 ml) and stirred at 0°C for an hour (during this time dicyclohexyl urea started separating an indication of activation) . Then a precooled miture of Asp (Bzl) Sar Lys (2Cl - Z) hydrochloride (20 g ; 0.028 m), triethyl amine (4 ml ; 0.028 m)and dry dichloromethane (100 ml) was added to the reaction mixture in lots. After the addition was complete , reaction mixture was stirred at 0°C for six hours , than at room temperature for 48 hours . The reaction mixture was cooled at - 5 °C for two hours and separated solid (dicyclohexyl urea) was filtered off under suction. Solvent from the filterate was distilled off on rotavapour (below 40 °C) and residue was taken up in water (1 litre) and extracted with ethyl acetate (1 litre). Organic layer was washed with 5 % NaHCO₃ solution 2 x 200 ml ; water 2 x 200ml; 1 N HCl 2 x 200ml ; water 2x 200 ml and dried over anhydrous sodium sulphate (200 g). After removal of salt by filteration the solvent from filtrate was distilled off on rotavapour (below 40 °C), residue was recrystallised from ethylacetate-ether. Yield 10.5 g (35 %) ; TLC (10 % Methanol-Chloroform) Rf : 0.65 ; HPLC : 96.25 % ; FAB Mass : 1000 .

**Ala-Gly-Gly-Asp-Sar-Lys . Diacetate :** Z Ala Gly Gly Asp (Bzl) Sar Lys (2Cl - Z)Bzl (9 g ; 0.09 m) was taken up in 25 % acetic acid- methanol (300 ml) and 10 % palladium on carbon (2.0 g) was added to it and a stream of hydrogen was bubbled into the reaction mixture at room temperature for four hours . The catalyst was filtered off and throughly washed with methanol . Solvent from the filterate was distilled off on rotavapour (below 40 °C) first on water vacuum and finally under high vacuum . Residue was teturated with dry ether separated solid was filtered , washed with dry ether and dried in a vacuum desicator . Finally this compound was purified by preparative HPLC. Yield 3.73 g (65 %) ; HPLC : 99 % ; FAB Mass: 518, 540 .

### Example - 9

Ten ml of 0.2 M phosphate buffer solution of pH 6.5 was prepared as per IP specifications, 40 mg of sodium chloride was added to it with proper shaking followed by addition of 5 mg of methyl cellulose. This solution was mixed well and 26 mg of the Ala-Gly-Gly-Asp-Sar-Lys was added under sonication to get the required solution.

### Example - 10

Ala-Gly-Gly-Asp-Sar-Lys (52 mg) and beta-cyclodextrin (113.5 mg) was added to 25 ml of water in a flask. The solution was left shaking at 40°C for 5 hrs to make a clear solution. This solution was frozen and then freeze dried. The free flowing Ala-Gly-Gly-Asp-Sar-Lys: beta-cyclodextrin complex (1:1) so obtained was washed with 10 ml methanol : chloroform (1:4) and dried. Ten ml of 0.2 M phosphate buffer solution of pH 6.5 was prepared as per IP specifications, 40 mg of sodium chloride was added to it with proper shaking followed by addition of 5 mg of methyl cellulose. This solution was mixed well and 83 mg of the Ala-Gly-Gly-Asp-Sar-Lys : beta-cyclodextrin complex (1:1) prepared earlier was added under stirring to get the required solution.

### Example - 11

Ala-Gly-Gly-Asp-Sar-Lys (52 mg) and alpha-cyclodextrin (97.3 mg) was added to 25 ml of water in a flask. The solution was left shaking at 40° C for 5 hrs to make a clear solution. This solution was frozen and then freeze dried. The free flowing Ala-Gly-Gly-Asp-Sar-Lys : alpha-cyclodextrin complex (1:1) so obtained was washed with 10 ml methanol : chloroform (1:4) and dried. Ten ml of 0.2 M phosphate buffer solution of pH 6.5 was prepared as per IP specifications, 40 mg of sodium chloride was added to it with proper shaking followed by addition of 5 mg of methyl cellulose. This solution was mixed well and 75 mg of the Ala-Gly-Gly-Asp-Sar-Lys : beta-cyclodextrin complex (1:1) prepared earlier was added under stirring to get the required solution.

### Example - 12

Ala-Asp-Ser-Asp-Sar-Lys (52 mg) and hydroxypropyl beta-cyclodextrin (260 mg) was added to 25 ml of water in a flask. The solution was left shaking at 40° C for 5 hrs to make a clear solution. This solution was mixed with dichlorodifluoro methane as propellent and filled in a stainless steel vial sealed with a metering valve and special nasal adapter.

## Claims

1. A polypeptide of formula 1: 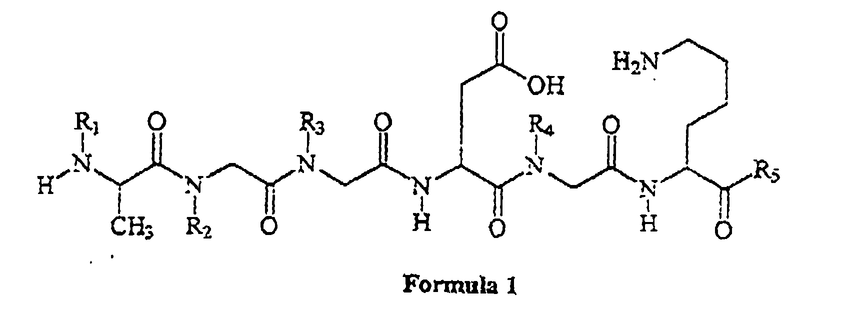 wherein R₁, R_{2,} R₃, R₄ are selected from the group consisting of H, CH₃ and CH₂=CH₂-CH₂ and R₅ is selected from the group consisting of OH, NH₂ and NHCₙH₂ₙ₊₁ (n=1 to 18), **characterised in that** the polypeptide contains unsubstituted amino acid residues and substituted amino acid residues, wherein each such substitution consists of either a methyl or an allyl group.

2. A polypepetide as claimed in claim 1 wherein the polypeptide is a hexapeptide selected from the group consisting of:
(a) Ala-Sar-Gly-Asp-Gly-Lys-OH
(b) N-MeAla-Gly-Sar-Asp-Gly-Lys-OH
(c) Ala-Sar-Sar-Asp-Gly-Lys_OH
(d) N-allylAla-Gly-Sar-Asp-Sar-Lys-OH
(e) Ala-Sar-Gly-Asp-Sar-Lys-OH
(f) Ala-Gly-Gly-Asp-Sar-Lys-NH₂
(g) Ala-Gly-Sar-Asp-Sar-Lys-NHPr(n)
(h) Ala-Gly-Gly-Asp-Sar-Lys-NH₂
(i) Ala-Gly-Gly-Asp-Sar-Lys-OH

3. A polypeptide as claimed in claim 1 wherein the R₁ = H; R₂ = CH₃; R₃ = H; R₄ = CH₃ (i.e. the sequence is Ala-Sar-Gly-Asp-Sar-Lys) and R₅ is OH or amide or amide group substituted with aliphatic chains.

4. A pharmaceutical composition comprising a polypeptide of formula 1: 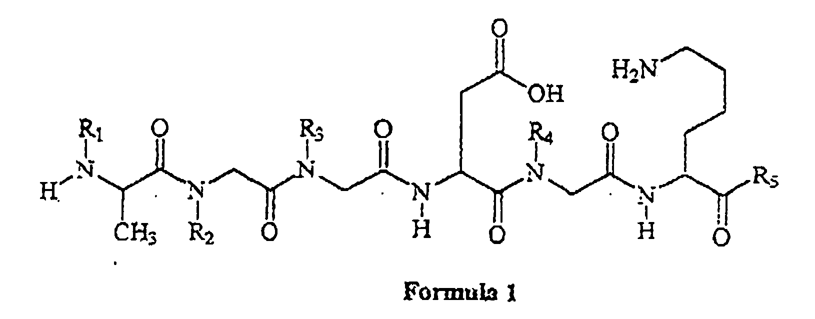 wherein R₁, R₂, R₃, R₄ are selected from the group consisting of H, CH₃ and CH₂=CH₂-CH₂ and R₅ is selected from the group consisting of OH, NH₂ and NHCₙH₂ₙ₊₁ (n=1 to 18) in admixture with a pharmaceutically acceptable carrier, **characterised in that** the polypeptide contains unsubstituted amino acid residues and substituted amino acid residues, wherein each such substitution consists of either a methyl or an allyl group.

5. A process for converting a polypeptide of formula 1: 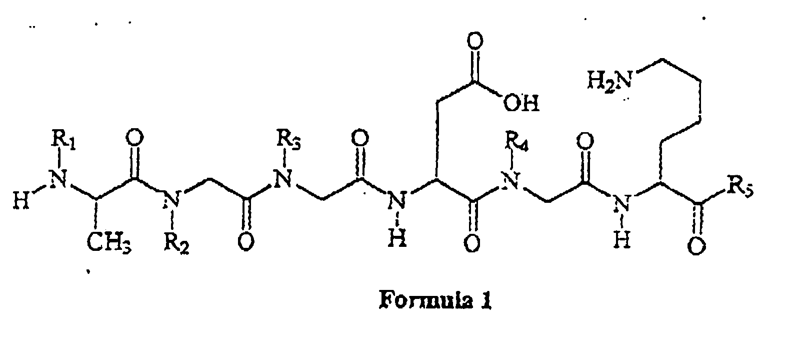 wherein R₁, R₂, R₃, R₄ are selected from the group consisting of H, CH₃ and CH₂=CH₂-CH₂ and R₅ is selected from the group consisting of OH, NH₂ and NHCₙH₂ₙ₊₁ (n=1 to 18) to a pharmaceutically acceptable formulation, comprising preparing solutions of said peptide and cyclodextrin in a protic solvent separately, mixing the above said solutions at a temperature in the range of 10 to 80°C to make a clear solution, removing the solvent to get a free flowing complex, mixing the complex so obtained in a vehicle to get the said formulation, wherein the polypeptide contains unsubstituted amino acid residues and substituted amino acid residues, wherein each such substitution consists of either a methyl or an allyl group.

6. A process as claimed in claim 5 wherein the cyclodextrin used is selected from the group consisting of naturally occurring alpha-cyclodextrin, beta-cyclodextrin, gamma-cyclodextrin and their derivatives selected in turn from the group consisting of dimethyl beta-cyclodextrin and hydroxy propyl beta-cyclodextrin.

7. A process as claimed in claim 5 wherein the solvent is removed by freeze drying, spray drying, coprecipitation or solvent evaporation.

8. A process as claimed in claim 5 wherein the vehicle used is selected from the group consisting of 0.2 M phosphate buffer solution of pH 6.5 containing sodium chloride and methyl cellulose, and a mixture of alcohol and commercial propellant.

9. A process as claimed in claim 5 wherein the amount of hexapeptide used ranges from 5 to 40% by weight of the inclusion complex [1:5 to 1:1].

10. A process as claimed in claim 5 where in the formulation is made in the form of nasal drops/spray.

11. The use of a polypeptide of formula 1: 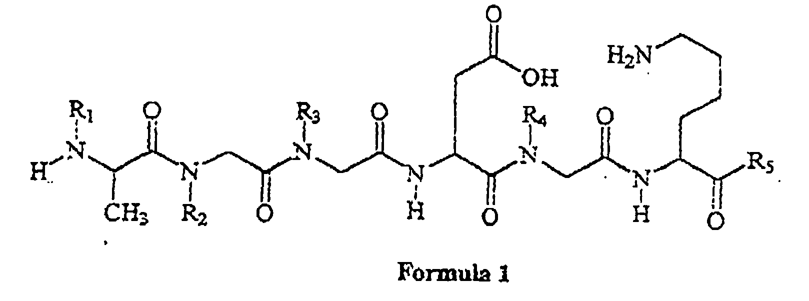 wherein R₁, R₂, R₃, R₄ are selected from the group consisting of H, CH₃ and CH₂=CH₂-CH₂ and R₅ is selected from the group consisting of OH, NH₂ and NHCₙH₂ₙ₊₁ (n=1 to 18) in the manufacture of a composition for the treatment of allergy/asthma disorders, wherein the polypeptide contains unsubstituted amino acid residues and substituted amino acid residues, wherein each such substitution consists of either a methyl or an allyl group.

12. The use as claimed in claim 11 wherein the amount of said composition to be administered to said subject is in the range of 0.5 to 5.0 mg/kg of body weight of the subject.

13. The use as claimed in claim 11 wherein the pharmaceutical composition is adapted for administration to said subject as a nasal formulation.

## Patentansprüche

1. Polypeptid der Formel 1: 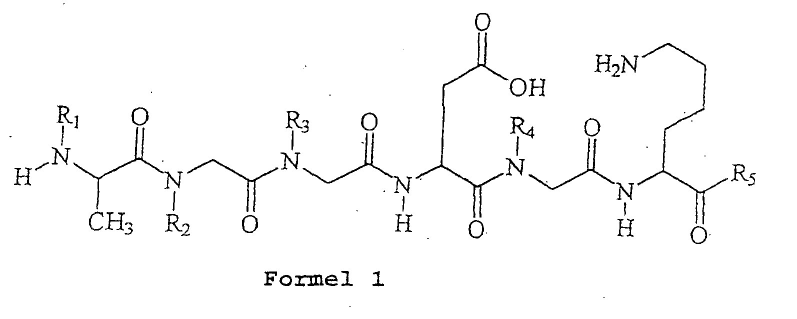 worin R₁,R₂, R₃ und R₄ ausgewählt sind aus der Gruppe, bestehend aus H, CH₃ und CH₂=CH₂-CH₂, und R₅ ausgewählt ist aus der Gruppe, bestehend aus OH, NH₂ und NHCₙH₂ₙ₊₁ (n= 1-18), **dadurch gekennzeichnet, dass** das Polypeptid unsubstituierte Aminosäurereste und substituierte Aminosäurereste enthält, wobei jede Substitution entweder aus einer Methyl- oder einer Allylgruppe besteht.

2. Polypeptid nach Anspruch 1, wobei das Polypeptid ein Hexapeptid ist, ausgewählt aus der Gruppe, bestehend aus:
(a) Ala-Sar-Gly-Asp-Gly-Lys-OH
(b) N-MeAla-Gly-Sar-Asp-Gly-Lys-OH
(c) Ala-Sare-Sar-Asp-Gly-Lys-OH
(d) N-allylAla-Gly-Sar-Asp-Sar-Lys-OH
(e) Ala-Sar-Gly-Asp-Sar-Lys-OH
(f) Ala-Gly-Gly-Asp-Sar-Lys-NH₂
(g) Ala-Gly-Sar-Asp-Sar-Lys-NHPr(n)
(h) Ala-Gly-Gly-Asp-Sar-Lys-NH₂
(i) Ala-Gly-Gly-Asp-Sar-Lys-OH.

3. Polypeptid nach Anspruch 1, worin R₁ = H, R₂ = CH₃, R₃ = CH₃ und R₄ = CH₃ (d.h. die Sequenz Ala-Sar-Gly-Asp-Sar-Lys) und R₅ OH oder ein Amid oder eine mit aliphatischen Ketten substituierte Amidgruppe ist.

4. Pharmazeutische Zubereitung, die ein Polypeptid der Formel 1 umfasst, 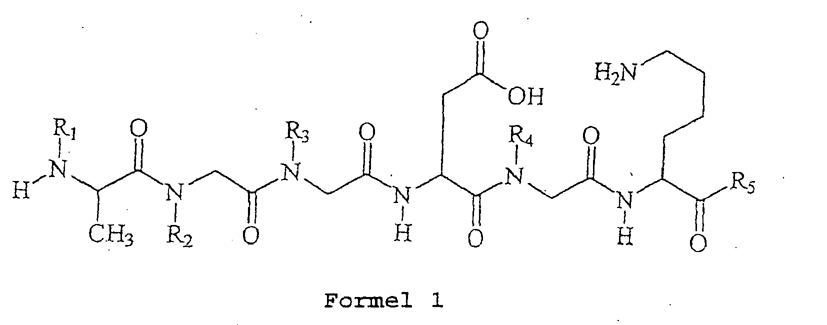 worin R₁, R₂, R₃ und R₄ ausgewählt sind aus der Gruppe, bestehend aus H, CH₃ und CH₂=CH₂-CH₂, und R₅ ausgewählt ist aus der Gruppe, bestehend aus OH, NH₂ und NHCₙH₂ₙ₊₁ (n= 1-18), im Gemisch mit einem pharmazeutisch verträglichen Träger, **dadurch gekennzeichnet, dass** das Polypeptid unsubstituierte Aminosäurereste und substituierte Aminosäurereste enthält, wobei jede Substitution entweder aus einer Methyl- oder einer Allylgruppe besteht.

5. Verfahren zur Umwandlung eines Polypeptids der Formel 1: 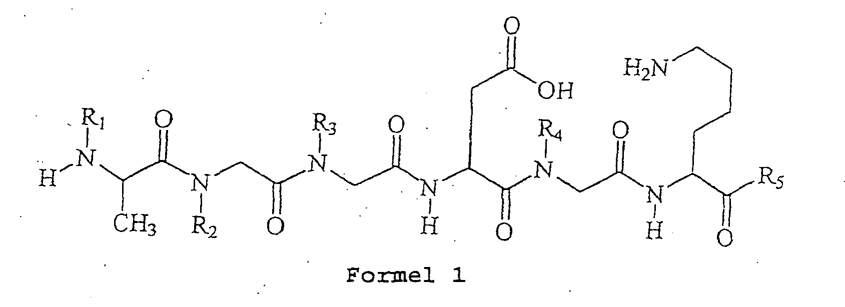 worin R₁, R₂, R₃ und R₄ ausgewählt sind aus der Gruppe, bestehend aus H, CH₃ und CH₂=CH₂-CH₂, und R₅ ausgewählt ist aus der Gruppe, bestehend aus OH, NH₂ und NHCₙH₂ₙ₊₁ (n= 1-18), in eine pharmazeutisch verträgliche Formulierung, welche die Herstellung von Lösungen des Peptids und getrennt davon von Cyclodextrin in einem protischen Lösungsmittel, das Mischen der obigen Lösungen bei einer Temperatur im Bereich von 10 bis 80°C unter Gewinnung einer klaren Lösung, die Entfernung des Lösungsmittels zur Gewinnung eines frei fließenden Komplexes und das Mischen des auf diese Weise erhaltenen Komplexes in einem Vehikel unter Bildung der genannten Formulierung umfasst, worin das Polypeptid unsubstituierte Aminosäurereste und substituierte Aminosäurereste enthält, wobei jede Substitution entweder aus einer Methyl- oder einer Allylgruppe besteht.

6. Verfahren nach Anspruch 5, bei dem das verwendete Cyclodextrin ausgewählt wird aus der Gruppe, bestehend aus natürlich vorkommendem α-, β- und γ-Cyclodextrin und ihren Derivaten, die ihrerseits ausgewählt werden aus der Gruppe, bestehend aus Dimethyl-β-cyclodextrin und Hydroxypropyl-β-cyclodextrin.

7. Verfahren nach Anspruch 5, bei dem das Lösungsmittel durch Gefriertrocknung, Sprühtrocknung, Kopräzipitation oder Lösungsmittelverdampfung entfernt wird.

8. Verfahren nach Anspruch 5, bei dem das verwendete Vehikel ausgewählt wird aus der Gruppe, bestehend aus 0,2 M Phosphatpufferlösung bei einem pH von 6,5, die Natriumchlorid und Methylcellulose enthält, und einem Gemisch aus Alkohol und handelsüblichem Treibgas.

9. Verfahren nach Anspruch 5, bei dem die Menge an Hexapeptid in einem Bereich von 5 bis 40 Gew.-% der Einschlussverbindung [1:5 bis 1:1] liegt.

10. Verfahren nach Anspruch 5, bei dem die Formulierung in Form von Nasentropfen bzw. eines Nasensprays hergestellt wird.

11. Verwendung eines Polypeptids der Formel 1: 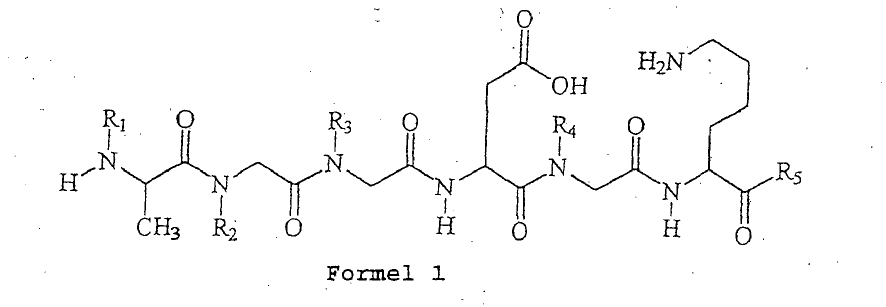 worin R₁,R₂, R₃ und R₄ ausgewählt sind aus der Gruppe, bestehend aus H, CH₃ und CH₂=CH₂-CH₂, und R₅ ausgewählt ist aus der Gruppe, bestehend aus OH, NH₂ und NHCₙH₂ₙ₊₁ (n= 1-18), bei der Herstellung einer Zusammensetzung zur Behandlung von allergischen und asthmatischen Störungen, worin das Polypeptid unsubstituierte Aminosäurereste und substituierte Aminosäurereste enthält, wobei jede Substitution entweder aus einer Methyl- oder einer Allylgruppe besteht.

12. Verwendung nach Anspruch 11, wobei die Menge der dem Patienten zu verabreichenden Zubereitung in einem Bereich von 0,5-5,0 mg/kg Körpergewicht des Patienten liegt.

13. Verwendung nach Anspruch 11, wobei die pharmazeutische Zubereitung für die Verabreichung an den Patienten als nasale Formulierung angepasst ist.

## Revendications

1. Polypeptide de formule I : 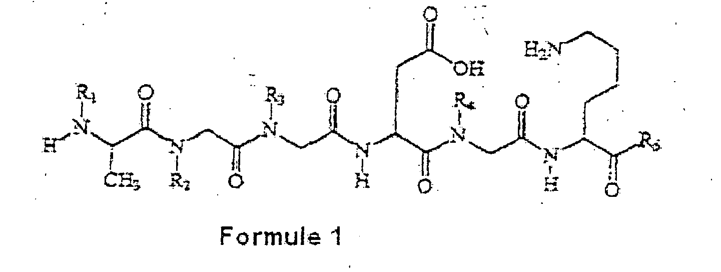 dans laquelle R₁, R₂, R₃, R₄ sont choisis dans le groupe constitué par H, CH₃, et CH₂=CH₂-CH₂ et R₅ est choisi dans le groupe constitué par OH, NH₂ et NHCₙH₂ₙ₊₁ (n = 1 à 18), **caractérisé en ce que** le polypeptide contient des résidus d'acides aminés non substitués et des résidus d'acides aminés substitués, où chaque substitution de ce type consiste soit en un groupe éthyle soit en un groupe allyle.

2. Polypeptide selon la revendication 1; dans lequel le polypeptide est un hexapeptide choisi dans le groupe constitué par:
(a) Ala-Sar-Gly-Asp-Gly-Lys-OH
(b) N-MeAla-Gly-Sar-Asp-Gly-Lys-OH
(c) Ala-Sar-Sar-Asp-Gly-Lys_OH
(d) N-allylAla-Gly-Sar-Asp-Sar-Lys-OH
(e) Ala-Sar-Gly-Asp-Sar-Lys-OH
(f) Ala-Gly-Gly-Asp-Sar-Lys-NH₂
(g) Ala-Gly-Sar-Asp-Sar-Lys-NHPr(n)
(h) Ala-Gly-Gly-Asp-Sar-Lys-NH₂
(i) Ala-Gly-Gly-Asp-Sar-Lys-OH

3. Polypeptide selon la revendication 1, dans lequel R₁ = H, R₂ = CH₃ ; R₃ = H ; R₄ = CH₃ (c'est-à-dire, la séquence est Ala-Sar-Gly-Asp-Sar-Lys) et R₅ est OH ou un groupe amide éventuellement substitué par des chaînes aliphatiques.

4. Composition pharmaceutique comprenant un polypeptide de formule I : 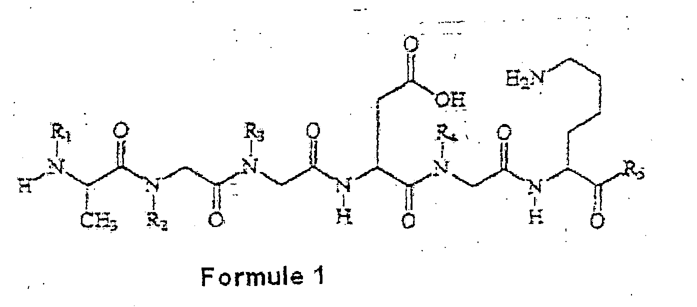 dans laquelle R₁, R₂, R₃, R₄ sont choisis dans le groupe constitué par H, CH₃, et CH₂=CH₂-CH₂ et R₅ est choisi dans le groupe constitué par OH, NH2 et NHCₙH₂ₙ₊₁ (n = 1 à 18) en mélange avec un véhicule pharmaceutiquement acceptable, **caractérisé en ce que** le polypeptide contient des résidus d'acides aminés non substitués et- des résidus d'acides aminés substitués, où chaque substitution de ce type consiste soit en un groupe éthyle soit en un groupe allyle.

5. Procédé de conversion d'un polypeptide de formule I : 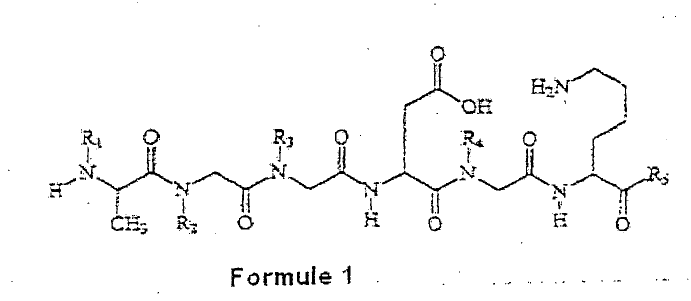 dans laquelle R₁, R₂, R₃, R₄ sont choisis dans le groupe constitué par H, CH₃, et CH₂=CH₂-CH₂ et R₅ est choisi dans le groupe constitué par OH, NH₂ et NHCₙH₂ₙ₊₁ (n = 1 à 18) en une formulation pharmaceutiquement acceptable, comprenant la préparation de façon séparée de solutions dudit peptide et de cyclodextrine dans un solvant protique, le mélange desdites solutions précitées à une température comprise dans la plage de 10 à 80°C pour obtenir une solution claire, l'élimination du solvant pour obtenir un complexe fluide, le mélange du complexe ainsi obtenu avec un véhicule pour obtenir ladite formulation, dans lequel le polypeptide contient des résidus d'acides aminés non substitués et des résidus d'acides aminés substitués, où chaque substitution de ce type consiste soit en un groupe éthyle soit en un groupe allyle.

6. Procédé selon la revendication 5, dans lequel la cyclodextrine utilisée est choisie dans le groupe constitué par la cyclodextrine α, la cyclodextrine β, la cyclodextrine γ existant à l'état naturel et leurs dérivés choisis à leur tour dans le groupe constitué par la diméthylcyclodextrine β et l'hydroxypropylcyclodextrine β.

7. Procédé selon la revendication 5, dans lequel le solvant est éliminé par lyophilisation, séchage par pulvérisation, coprécipitation ou évaporation de solvant.

8. Procédé selon la revendication 5, dans lequel le véhicule utilisé est choisi dans le groupe constitué par une solution de tampon phosphate 0,2 M de pH 6,5 contenant du chlorure de sodium et de la méthylcellulose, et un mélange d'alcool et d'agent propulseur du commerce.

9. Procédé selon la revendication 5, dans lequel la quantité d'hexapeptide utilisée varie de 5 à 40% en poids du complexe d'inclusion [1:5 à 1:1].

10. Procédé selon la revendication 5, où la formulation est préparée sous forme de gouttes/aérosol intranasal.

11. Utilisation d'un polypeptide de formule 1 : 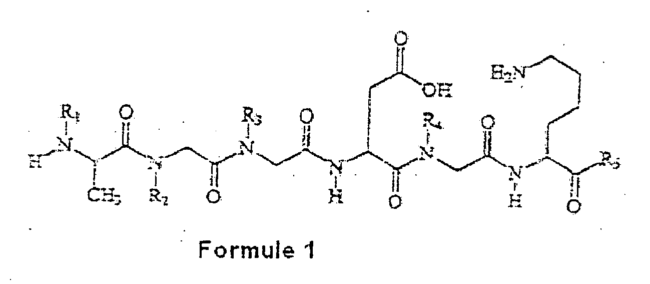 dans laquelle R₁, R₂, R₃, R₄ sont choisis dans le groupe constitué par H, CH₃, et CH₂=CH₂-CH₂ et R₅ est choisi dans le groupe constitué par OH, NH2 et NHCₙH₂ₙ₊₁ (n =1 à 18) dans la fabrication d'une composition pour le traitement de troubles allergiques/asthmatiques, **caractérisée en ce que** le polypeptide contient des résidus d'acides aminés non substitués et des résidus d'acides aminés substitués, où chaque substitution de ce type consiste soit en un groupe éthyle soit en un groupe allyle.

12. Utilisation selon la revendication 11, dans laquelle la quantité de ladite composition devant être administrée audit sujet est comprise dans la gamme de 0,5 à 5,0 mg/kg de poids corporel du sujet.

13. Utilisation selon la revendication 11, dans laquelle la composition pharmaceutique est d'administration adaptée audit sujet sous forme d'une formulation intranasale.
